(19) Europäisches Patentamt
European Patent Office
Office européen des brevets

(11) EP 2 962 709 B1

(12) **EUROPEAN PATENT SPECIFICATION**

(45) Date of publication and mention of the grant of the patent:
**06.12.2017 Bulletin 2017/49**

(21) Application number: **13876722.3**

(22) Date of filing: **26.02.2013**

(51) Int Cl.:
***A61M 1/02*** (2006.01) ***A61M 1/36*** (2006.01)
***B04B 13/00*** (2006.01)

(86) International application number:
**PCT/JP2013/054881**

(87) International publication number:
**WO 2014/132327 (04.09.2014 Gazette 2014/36)**

(54) **BLOOD COMPONENT SEPARATION APPARATUS**

VORRICHTUNG ZUR TRENNUNG VON BLUTKOMPONENTEN

APPAREIL DE SÉPARATION DES COMPOSANTS DU SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.01.2016 Bulletin 2016/01**

(73) Proprietor: **Terumo Kabushiki Kaisha**
**Tokyo 151-0072 (JP)**

(72) Inventor: **KIMURA, Shigeyuki**
**Fujinomiya-shi**
**Shizuoka 418-0004 (JP)**

(74) Representative: **Casalonga**
**Casalonga & Partners**
**Bayerstraße 71/73**
**80335 München (DE)**

(56) References cited:
**EP-A1- 0 580 299**
**WO-A1-2014/041599**
**JP-A- H09 313 599**
**JP-A- 2002 126 072**
**JP-A- 2002 291 872**
**JP-A- 2009 226 210**
**JP-B2- 2 907 689**
**JP-B2- 3 196 838**
**JP-B2- 3 936 132**
**JP-B2- 4 848 125**
**US-B1- 6 743 192**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 962 709 B1

## Description

Technical Field

The present invention relates to a blood component separation device for collecting a predetermined blood component from blood.

Background Art

Conventionally, in the field of blood sampling, a blood component, such as platelet liquid, is collected by collecting only the component from sampled blood and returning the remaining blood components to the blood donor. In such an operation, a blood component separation device including a centrifugal separator is used.

In recent years, in the field of radiation therapy for cancer or the like, transfusion of platelet liquid has been widely performed, and high-concentration platelet liquid has become necessary for the therapy. To obtain high-concentration platelet liquid, Patent Literature 1 discloses an art using a blood component separation device to temporarily store low-concentration platelet liquid in a buffy coat bag and store only high-concentration platelet liquid in a platelet intermediate bag. Patent Literature 2 provides an apheresis machine and a method for producing a blood product from collected whole blood wherein the volume of whole blood to be processed in a centrifuge during a "draw" step is variably controlled in response to at least one characteristic, such as the number of platelets or hematocrit value, associated with the whole blood collected or to be collected from a donor.

Citation List

Patent Literature

Patent Literature 1: JP 2009-226210 A

Patent Literature 2: US 6,743,192 B1

Summary of Invention

Technical Problem

To surely collect platelet liquid satisfying a target concentration, an extracorporeally circulating volume of platelet liquid (sampled blood volume) during collecting operation should properly be adjusted. A technique of adjusting the extracorporeally circulating volume taking the hematocrit value of blood in account is not disclosed in Patent Literature 1.

To adjust the extracorporeally circulating volume taking the hematocrit value of blood in account, the hematocrit value of blood may be measured with a blood cell counter (blood cell counting device).

However, if there is a large difference between the hematocrit value measured with a blood cell counter and the actual hematocrit value, in other words, if the measuring accuracy of the blood cell counter is low, the collected platelet liquid may not satisfy the target concentration.

An example case will be explained where a hematocrit value measured with a blood cell counter is 40% while the actual hematocrit value is 45%. The hematocrit value measured with the blood cell counter is smaller than the actual hematocrit value. The target extracorporeally circulating volume of platelet liquid per one cycle during the collecting operation required of the blood component separation device is, for example, 450 ml according to calculation from the hematocrit value (40%) measured with the blood cell counter. Since the actual hematocrit value is 45%, which is different from the hematocrit value measured with the blood cell counter, the actual extracorporeally circulating volume per one cycle may be adjusted to 400 ml. When the actual extracorporeally circulating volume is significantly smaller than the target extracorporeally circulating volume, the concentration of the collected platelet liquid may be lower than the target concentration (unit shortage of platelets).

In another case, where the hematocrit value measured with a blood cell counter is larger than the actual hematocrit value, the concentration of collected platelet liquid may be higher than the target concentration (unit excess of platelets).

As described in these examples, the collected platelet liquid may not satisfy the target concentration due to the low measuring accuracy of the blood cell counter.

The present invention is made to solve the aforementioned problem. The object of the present invention is to provide a blood component separation device capable of collecting a predetermined blood component to satisfy a target concentration regardless of the measuring accuracy of the blood cell counter.

Solution to Problem

The invention is defined by the appended claims.

To solve the problem, one aspect of the present invention is a blood component separation device which includes a centrifugal separator configured to separate a plurality of blood components from blood and a container for containing a predetermined blood component centrifugally separated, and which is configured to perform a plurality of steps of collecting the predetermined blood component which is separated, and the blood component separation device includes a calculation unit configured to calculate an estimated extracorporeal circulation volume as a target sampled blood volume per one cycle of an operation of collecting the predetermined blood component from a hematocrit value obtained by correcting a hematocrit value measured with a blood cell counter. The calculation unit is configured to calculate a correction value used for the correction from a data value of the estimated extracorporeal circulation volume of a plurality of finished and immediately preceding collecting operations and a data value of sampled blood volume measured in a first cycle of each of a plurality of immediately preceding collecting operations.

According to the aspect, a correction value for correcting a measured hematocrit value is calculated from a data value of a plurality of finished and immediately preceding operations of collecting a predetermined blood component. The correction value is used for correcting the measured hematocrit value to calculate an estimated extracorporeal circulation volume of the present collecting operation. By correcting the hematocrit value measured with the blood cell counter, the blood component satisfying the target concentration can be collected even if the measuring accuracy of the blood cell counter is low. The correction value for correcting the measured hematocrit value is updated upon finishing each collecting operation, so that the measured hematocrit value is suitably corrected along with the change in the measuring accuracy of the blood cell counter. Therefore the blood component satisfying the target concentration can be collected regardless of the accuracy of measuring the hematocrit value of the blood cell counter.

According to the aspect, a data value of the estimated extracorporeal circulation volume and a data value of sampled blood volume measured in the first cycle are preferably calculated without using the data value of a collecting operation in which blood sampling is stopped before finishing the collecting operation.

According to the aspect, the correction value for correcting measured hematocrit value is calculated using only the data value of a collecting operation which succeeded in collecting the predetermined blood component. Thus the accuracy of correcting the measured hematocrit value improves and the estimated extracorporeal circulation volume can further accurately be calculated. Therefore the platelet liquid satisfying the target concentration can surely be collected regardless of the accuracy of measuring the hematocrit value of the blood cell counter.

Furthermore, according to the aspect, the blood component separation device is preferably configured to perform a) a centrifugal separation step of introducing whole blood drawn from a blood donor into the centrifugal separator to separate whole blood into a plurality of blood components, b) a circulation flow step of introducing a first blood component, among predetermined blood components separated in the centrifugal separation, into the centrifugal separator together with whole blood, c) a circulation/acceleration step, performed after a predetermined volume of the first blood component is separated in the circulation flow step, of stopping the supply of whole blood to the centrifugal separator to introduce only the first blood component into the centrifugal separator, further performing circulation for a predetermined period of time, and then increasing the circulation speed so that a second blood component is separated in the centrifugal separator and collected, and d) a blood returning step, performed after collecting a predetermined volume of the second blood component in the circulation/acceleration step, of returning blood components, which are not collected, to the blood donor. A cycle from the steps a) to d) is preferably performed a plurality of times.

According to the aspect, the predetermined blood component can accurately be separated from other blood components.

Furthermore, in the aspect, the circulation/acceleration step includes a first collecting step of transferring a portion of the second blood component with low-concentration among the second blood components to a temporary storage container and a second collecting step of collecting a portion of the second blood component with high-concentration among the second blood components. The second blood component with low-concentration transferred to the temporary storage container may preferably be introduced into the centrifugal separator together with the whole blood sampled in the following cycle.

According to the aspect, such a process can be used for BC recycling to obtain the second blood component with high-concentration, and thereby further larger volume of predetermined blood component can be collected.

In the aspect, it is preferable that the predetermined blood component is platelet liquid.

According to the aspect, the platelet liquid satisfying the target concentration can be collected regardless of the accuracy of measuring the hematocrit value of the blood cell counter.

Advantageous Effects of Invention

According to the blood component separation device configured as described above, the predetermined blood component satisfying the target concentration can be collected regardless of the measuring accuracy of the blood cell counter.

Brief Description of Drawings

Fig. 1 illustrates a configuration of a blood component separation device according to a first example.

Fig. 2 is a block diagram illustrating a control system of the blood component separation device according to an embodiment.

Fig. 3 illustrates a structure of a centrifuge bowl.

Fig. 4 is a flow chart illustrating operation of the blood component separation device according to the first example.

Fig. 5 is a flow chart illustrating operation performed in a collecting step of collecting platelet liquid.

Fig. 6 illustrates a first step (starting blood sampling step) of the blood component separation device according to the first example.

Fig. 7 illustrates a second step (centrifugal separation step).

Fig. 8 illustrates a third step (critical flow step).

Fig. 9 illustrates a circulation step in a fourth step (circulation/acceleration step).

Fig. 10 illustrates a step of collecting low-concentration platelet liquid performed in the fifth step (circulation/acceleration step).

Fig. 11 illustrates a step of storing high-concentration platelet liquid performed in the fifth step (circulation/acceleration step).

Fig. 12 illustrates a step of collecting low-concentration platelet liquid performed in the fifth step (circulation/acceleration step).

Fig. 13 illustrates a blood returning step.

Fig. 14 illustrates the first step performed in a second cycle.

Fig. 15 illustrates a second step performed in the second cycle.

Fig. 16 illustrates the third step performed in the second cycle.

Fig. 17 illustrates a processing step of platelet liquid.

Fig. 18 illustrates a final processing step of platelet liquid.

Fig. 19 illustrates operation of the blood component separation device in a chronological order.

Fig. 20 illustrates changes in concentrations of platelets, white blood cells, and red blood cells flowing out.

Fig. 21 illustrates an example of a data value of estimated extracorporeal circulation volume and a data value of drawn volume of blood measured for each cycle.

Fig. 22 illustrates a configuration of a blood component separation device according to a second example.

Fig. 23 is a flow chart illustrating an operation of the blood component separation device according to the second example.

Fig. 24 illustrates a blood sampling step of the blood component separation device according to the second example.

Fig. 25 illustrates a circulation step of the blood component separation device according to the second example.

Fig. 26 illustrates a PC collecting step of the blood component separation device according to the second example.

Description of Embodiments

Now, an embodiment of the blood component separation device according to the present invention will be described in detail referring to the drawings.

<First Example>

Fig. 1 illustrates a system configuration of a blood component separation device according to a first example. Fig. 2 is a block diagram illustrating a control system of the blood component separation device according to an embodiment.

The blood component separation device according to the embodiment includes a blood component separation circuit 1. The blood component separation circuit 1 includes an initial flow blood collecting circuit 5 composed of a blood sampling needle 2, an initial flow blood collecting bag Y7 for collecting initial flow blood, a sampling port 3, and an initial flow blood collecting line 4.

The blood component separation circuit 1 includes a centrifuge bowl E1. The centrifuge bowl E1 includes a rotor (not shown) having therein a space for storing drawn blood, a rotor drive unit 14 for rotating the rotor, an inflow port (first port E1a), and an outflow port (second port E1b), and is configured to separate blood into a plurality of blood components by rotating the rotor. The blood component separation circuit 1 includes three containers for storing blood components separated in the centrifuge bowl E1, that is, a first container (plasma bag) Y1, a second container (temporary storage bag) Y2, and a third container (platelet intermediate bag) Y3.

The blood component separation circuit 1 includes a first line, a second line, a third line, a fourth line, a fifth line, a sixth line, and a seventh line.

The first line couples the blood sampling needle 2 and the centrifuge bowl E1 and includes a donor tube T1, a first blood pump P1, a tube T2, a tube T3a, a first open/close valve V1, a tube T3b, and a tube T4. The second line couples the centrifuge bowl E1 and the first container Y1 and includes a tube T5, a tube T6a, a second open/close valve V2, and a tube T6b. The third line couples the first container Y1 and the first line and includes a tube T8a, a third open/close valve V3, a tube T8b, a tube T9, a second blood pump P2, a tube T10b, a fourth open/close valve V4, and a tube T10a.

The fourth line couples the centrifuge bowl E1 and the second container Y2 and includes a tube T5, a tube T15, a tube T11a, a fifth open/close valve V5, and a tube T11b. The fifth line couples the second container Y2 and the first line and includes a tube T12, a tube T13b, a sixth open/close valve V6, and a tube T13a. The sixth line couples the second container Y2 and the first line, similarly to the fifth line, and includes a tube T12, a tube T14a, a seventh open/close valve V7, a tube T14b, a tube T9, the second blood pump P2, the tube T10b, the fourth open/close valve V4, and the tube T10a. The seventh line couples the centrifuge bowl E1 and the third container Y3 and includes the tube T5, the tube T15, a tube T16, a tube T17a, an eighth open/close valve V8, and a tube T17b.

The blood sampling needle 2 for sampling whole blood (blood) from a blood donor is coupled to the first port of the first blood pump P1 via the donor tube T1.

The initial flow blood collecting bag Y7 is coupled to the blood sampling needle via a branch provided on the donor tube T1 and via the initial flow blood collecting line 4. The initial flow blood collecting bag Y7 includes a sampling port 3 for transferring collected initial flow blood to a test container (not shown). The sampling port 3 is constituted with a main body, a needle 6, and a cover 7 for covering the needle 6. Further, a clamp 8 is provided on the initial flow blood collecting line 4 to open/close the line.

The tube T2 coupled to the second port of the first blood pump P1 is branched into the tube T3a and the tube T13a. The tube T3a is coupled to the first port of the first open/close valve V1, and the second port of the first open/close valve V1 is coupled to the tube T3b. The tube T3b is branched into the tube T4 and the tube T10a. The tube T4 is coupled to the first port E1a of the centrifuge bowl E1, which is a centrifugal separator for separating collected blood into a plurality of blood components. The centrifuge bowl E1 is disposed on the rotor drive unit 14 to be rotated.

The blood sampling needle 2 and the first port E1a, which is an inlet port of the centrifuge bowl E1, are coupled via the first line (the donor tube T1, the first blood pump P1, the tube T2, the tube T3a, the first open/close valve V1, the tube T3b, and the tube T4).

A pressure sensor C1 is coupled to the donor tube T1.

The tube T5 coupled to the second port E1b of the centrifuge bowl E1 is branched into the tube T15 and the tube T6a. The tube T6a is coupled to the first port of the second open/close valve V2, and the second port of the second open/close valve V2 is coupled to the tube T6b. The tube T6b is coupled to the second port Y1b of the plasma bag (the first container) Y1.

The second port E1b of the centrifuge bowl E1 and the plasma bag Y1 are coupled via the second line (the tube T5, the tube T6a, the second open/close valve V2, and the tube T6b). Two plasma bags Y1 are provided, though only one plasma bag is illustrated in Figs. 6 to 18.

The first port Y1a, or the outlet port, of the plasma bag Y1 is coupled to the tube T8a. The tube T8a is coupled to the first port of the third open/close valve V3. The second port of the third open/close valve V3 is coupled to the tube T8b, and the tube T8b is coupled to the tube T9. The tube T9 is coupled to the second port of the second blood pump P2. The first port of the second blood pump P2 is coupled to the tube T10b, and the tube T10b is coupled to the second port of the fourth open/close valve V4. The first port of the fourth open/close valve V4 is coupled to the tube T10a.

The tube T10a is coupled to the connection between the tube T3b and the tube T4 constituting the first line. The plasma bag Y1 and the first line are coupled via the third line (the tube T8a, the third open/close valve V3, the tube T8b, the tube T9, the second blood pump P2, the tube T10b, the fourth open/close valve V4, and the tube T10a). The plasma bag Y1 is thus configured to selectively communicate with the inlet port or the outlet port of the centrifuge bowl E1.

The tube T15 branched from the tube T5 branches into the tube T11a and the tube T16. The tube T11a is coupled to the first port of the fifth open/close valve V5, and the second port of the fifth open/close valve V5 is coupled to the tube T11b. The second port of the fifth open/close valve V5 is coupled to the second port Y2b of the temporary storage bag Y2 via the tube T11b. That is, the second port E1b of the centrifuge bowl E1 and the temporary storage bag Y2 are coupled via the fourth line (the tube T5, the tube T15, the tube T11a, the fifth open/close valve V5, and the tube T11b).

The first port Y2a of the temporary storage bag Y2 is coupled to the tube T12, and the tube T12 is branched into the tube T13b and the tube T14a. The tube T13b is coupled to the first port of the sixth open/close valve V6, and the second port of the sixth open/close valve V6 is coupled to the tube T13a. The tube T13a is coupled to the connection between the tube T2 and the tube T3a constituting the first line.

The tube T14a branched from the tube T12 is coupled to the first port of the seventh open/close valve V7, and the second port of the seventh open/close valve V7 is coupled to the tube T14b. The tube T14b is coupled to the connection between the tube T9 and the tube T8b, and the tube T9 is coupled to the second port of the second blood pump P2.

The first port of the second blood pump P2 is coupled to the tube T10b, and the tube T10b is coupled to the first port of the fourth open/close valve V4. The second port of the fourth open/close valve V4 is coupled to the tube T10a. The tube T10a is coupled to the connection between the tube T3b and the tube T4 constituting the first line. The temporary storage bag Y2 and the first line are coupled via the fifth line (the tube T12, the tube T13b, the sixth open/close valve V6, and the tube T13a) and the sixth line (the tube T12, the tube T14a, the seventh open/close valve V7, the tube T14b, the tube T9, the second blood pump P2, the tube T10b, the fourth open/close valve V4, and the tube T10a). The temporary storage bag Y2 is coupled so as to selectively communicate with the inlet port or the outlet port of the centrifuge bowl E1.

The tube T16 branched from the tube T15 branches into the tube T17a and the tube T18a. The tube T17a is coupled to the first port of the eighth open/close valve V8, and the second port of the eighth open/close valve V8 is coupled to the tube T17b. The tube T17b is coupled to the first port Y3a, or the inlet port, of the platelet intermediate bag (the third container) Y3. The tube T18a branched from the tube T16 is coupled to the first port of the ninth open/close valve V9, and the second port of the ninth open/close valve V9 is coupled to the tube T18b. The tube T18b is coupled to the air bag Y4. That is, the second port E1b of the centrifuge bowl E1 and the platelet intermediate bag Y3 are coupled via the seventh line (the tube T5, the tube T15, the tube T16, the tube T17a, the eighth open/close valve V8, and the tube T17b). The platelet intermediate bag Y3 is thus configured to communicate with the outlet port of the centrifuge bowl E1.

A turbidity sensor C2 for detecting the concentration of platelets and the pressure sensor C3 are attached to the tube T5 coupled to the second port E1b of the centrifuge bowl E1. The turbidity sensor C2 detects the turbidity, caused by platelets, of plasma flowing in the tube T5.

In the peripheral region of where the centrifuge bowl E1 is disposed, an interface sensor C4 for detecting the location of the interface of a buffy coat layer BC (see Fig. 3) formed in the centrifuge bowl E1 is attached.

The tube T19 coupled to the second port Y3b, or the outlet port, of the platelet intermediate bag Y3, is branched into the tube T20a and the tube T21. The tube T20a is coupled to the first port of the tenth open/close valve V10, and the second port of the tenth open/close valve V10 is coupled to the tube T20b. The tube T21 is coupled to the first port, or the output port, of the third blood pump P3. The second port, or the input port, of the third blood pump P3 is coupled to a platelet reserve liquid bottle through a sterilizing filter 9 and a bottle needle 10. The tube T20b is coupled to the platelet bag Y5 via a white blood cell removal filter 11. The air bag Y6 is coupled to the platelet bag Y5.

An output port of the ACD pump P4 is coupled to the donor tube T1. The input port of the ACD pump P4 is coupled to the output port of the sterilizing filter 12. The input port of the sterilizing filter 12 is coupled to the ACD storing bottle via a bottle needle 13.

As illustrated in Fig. 2, a controller 15 is configured with, for example, a microcomputer. The controller 15 is electrically coupled to the first blood pump P1, the second blood pump P2, the third blood pump P3, the ACD pump P4, the centrifuge bowl drive device 14, the pressure sensor C1, the turbidity sensor C2, the pressure sensor C3, the interface sensor C4, the first open/close valve V1, the second open/close valve V2, the third open/close valve V3, the fourth open/close valve V4, the fifth open/close valve V5, the sixth open/close valve V6, the seventh open/close valve V7, the eighth open/close valve V8, the ninth open/close valve V9, and the tenth open/close valve V10. The controller 15 is electrically coupled to a blood cell counter 32 which measures the hematocrit value of blood. The controller 15 is also electrically coupled to a processed blood volume measuring unit 34 which measures the drawn volume of blood (sampled blood volume).

The detection signals from the sensors C1, C2, C3, and C4 are input to the controller 15 as needed. Instructed by these detection signals, the controller 15 controls the pumps P1, P2, P3, and P4 to operate or stop, and controls the rotational direction (normal or reverse) and the rotational speed of the pumps. The controller 15 also controls the open/close valves V1, V2, V3, V4, V5, V6, V7, V8, V9, and V10 to open or close, and controls the centrifuge bowl drive device 14 to operate as required. The data of hematocrit value (HCT value) measured with the blood cell counter 32 and the data of drawn volume of blood measured with the processed blood volume measuring unit 34 are input to the controller 15 as needed. The controller 15 also functions as the "calculation unit" of the present invention.

As a material of the tubes, for example, thermoplastic elastomers such as polyvinyl chloride, polyethylene, polypropylene, polyester such as PET and PBT, ethylene-vinyl acetate copolymer (EVA), polyurethane, and polyester elastomer may be used. Among these materials, in particular, polyvinyl chloride is preferably used. Polyvinyl chloride not only has sufficient ductility and flexibility but also is easy to handle and suitable to be choked by a clamp or the like.

As a material of the bags, soft polyvinyl chloride including DEHP as a plasticizer or products of polymerization or copolymerization of such olefins or diolefins as polyolefin, ethylene, propylene, butadiene, and isoprene can be used. Typical examples include ethylene-vinyl acetate copolymer (EVA), polymer blends formed between EVA and various thermoplastic elastomers, and arbitrary combinations thereof. Alternatively, PET, PBT, PCGT, or the like can be used. Among these materials, particularly, polyvinyl chloride is preferably used. Such material having high gas permeability is preferable for a container for storing platelet liquid to improve shelf life of platelet liquid. Therefore, polyolefin or DnDp-plasticized polyvinyl chloride may preferably be used for such material or a material formed in a thin sheet may preferably be used.

Fig. 3 illustrates a structure of the centrifuge bowl E1. In Fig. 3, the figure is divided by the center line, where the right hand side illustrates a sectional view and the left hand side illustrates an external view in dashed lines.

The inflow port E1a and the outflow port E1b are formed on the non-rotating fixed portion 20 in the blood component separation device. The fixed portion 20 includes a cover 17 and an inflow tube 18 extending downward. These fixed portions rotatably and integrally support a side wall 21, an outer shell 22, an inner shell 23, and a bottom plate 16. The bottom plate 16 is coupled to the centrifuge bowl drive device 14 by suctioning so that the rotational force can be transferred from the centrifuge bowl drive device 14 to the bottom plate 16. Fig. 3 illustrates a state where whole blood is supplied into the centrifuge bowl E1 through the inflow port E1a and separated into blood components by centrifugal force.

That is, in the space between the outer shell 22 and the side wall 21 from the outer side to the inner side, in the descending order of specific gravity, a red blood cell layer RBC, a white blood cell layer WBC, a buffy coat layer BC, a platelet layer PLT, and a plasma layer PPP are formed by a centrifugal force. It is difficult to separate the white blood cell layer WBC and the platelet layer PLT, because the values of their specific gravities are close. Thus, the buffy coat layer BC including the white blood cell layer WBC and the platelet layer PLT is formed. Typically, the whole blood includes about 55% of plasma PPP, about 43.2% of red blood cells RBC, about 1.35% of white blood cells WBC, and 0.45% of platelets PLT.

The centrifuge bowl E1 has an outflow passage 19 in the inner periphery formed somewhat above the middle point of the inflow tube 18. So that the plasma layer PPP formed in the inner side of the space formed by the outer shell 22 and the side wall 21 flows out of the centrifuge bowl E1 through the outflow port E1b.

The operation of the blood component separation device configured as described above is illustrated in flow charts in Figs. 4 and 5. The operation and steps performed in the blood component separation device are illustrated in Figs. 6 to 18. The object of the device is to collect high-concentration platelet liquid. Fig. 19 is a processing drawing illustrating the operation of the blood component separation device in a chronological order.

Fig. 6 illustrates a starting blood sampling step (the first step). The pump outlined with a white inside shows that the pump is operating. The pump outlined with a black inside shows that the pump is not operating. The open/close valve outlined with a white inside shows that the valve is opened. The open/close valve outlined with a black inside shows that the valve is closed.

First, a priming step (S1) illustrated in Fig. 4 is performed. The ACD pump P4 and the first pump P1 are operated to supply ACD liquid, which prevents blood coagulation, to the centrifuge bowl E1 via the opened first open/close valve V1, thereby performing the priming step (S1) of the centrifuge bowl E1, the first blood pump P1, etc. The priming step is performed to previously apply the ACD liquid on portions in the donor tube T1, the first pump P1, the centrifuge bowl E1, etc., which are to make contact with blood, so that the blood does not coagulate when introduced. From the priming step, the centrifuge bowl drive device 14 rotates the centrifuge bowl E1 at a predetermined rotational speed.

When the priming step (S1) is finished, the blood sampling needle 2 pierces the blood donor to start drawing of whole blood (S2). After the blood sampling needle 2 piercing the blood donor, the initial flow blood is collected in the initial flow blood collecting bag Y7 of the initial flow blood collecting circuit (see Fig. 1). The branch provided on the donor tube T1 is initially configured to couple the blood sampling needle 2 and the initial flow blood collecting line 4 (see Fig. 1). When a predetermined volume of blood is stored in the initial flow blood collecting bag, the initial flow blood collecting line 4 is choked by the clamp 8 (see Fig. 1) to secure a flow passage toward the first blood pump P1 in the donor tube T1.

The ACD pump P4 is operated again to supply the ACD liquid to the donor tube T1 so that the ACD liquid is mixed with the whole blood, which is then supplied to the centrifuge bowl E1. When whole blood is supplied to the rotating centrifuge bowl E1, the air inside the centrifuge bowl E1 (shown in dashed lines) is pushed by the plasma to flow out through the outflow passage 19 (see Fig. 3) located in the inner periphery of the centrifuge bowl E1, as illustrated in Fig. 6. The air then flows through the opened ninth open/close valve V9 and is stored in the air bag Y4.

In the centrifuge bowl E1, as illustrated in Fig. 3, the supplied whole blood is separated into components by the centrifugal force generated in the bowl.

Then when the turbidity sensor C2 detects that the fluid flowing in the tube has changed from air to plasma, the ninth open/close valve V9 is closed and the second open/close valve V2 is opened to store the plasma spilled out of the centrifuge bowl E1 in the plasma bag Y1, as illustrated in Fig. 7. The centrifugal separation step (S3) is thus performed. As illustrated in Fig. 3, first, only plasma comes out of the centrifuge bowl E1.

Then when a certain volume of plasma (30 ml for the example) is stored in the plasma bag Y1 (S4: YES), the third open/close valve V3 is opened, the second blood pump P2 is operated, and the fourth open/close valve V4 is opened to draw whole blood from the blood donor, mix the whole blood with the plasma stored in the plasma bag Y1, and supply the mixture of the whole blood and the plasma to the centrifuge bowl E1, as illustrated in Fig. 8. A third step (critical flow step) (S5) is thus performed. These are performed in a critical flow period TE shown in Fig. 19.

When the interface sensor C4 detects that the interface between the buffy coat layer BC and the red blood cell layer RBC in Fig. 3 has come to a predetermined position (S6: YES), the first open/close valve V1 is closed with the second open/close valve V2, the third open/close valve V3, and the fourth open/close valve V4 opened, and the second blood pump P2 is kept operating as illustrated in Fig. 9. The plasma in the plasma bag Y1 flows through the third open/close valve V3, the second blood pump P2, the fourth open/close valve V4, the centrifuge bowl E1, and the second open/close valve V2 to return to the plasma bag Y1. A circulation step (fourth step) in the circulation/acceleration step is thus performed. This is performed in a circulation period TF shown in Fig. 19.

At the same time, whether the present cycle is the last cycle is determined. When the present cycle is not the last cycle (S7: NO), the sixth open/close valve V6 is opened, with the first blood pump P1 kept operating, to store the drawn whole blood in the temporary storage bag Y2 (S11). In other words, the drawing of whole blood can be continued by storing the drawn whole blood in the temporary storage bag Y2. Drawing of whole blood is continued until the circulation/acceleration step is finished, a predetermined time has elapsed, or a predetermined volume has been drawn. In the last cycle (S7: YES), the first blood pump P1 stops operating to stop blood drawing (S8).

In the circulation step in the circulation/acceleration step of the present example, the circulation speed is set higher than that of the critical flow step so that the plasma circulates at a speed of about 100 ml/min, thereby flowing through the centrifuge bowl E1 within 30 to 40 seconds. In this manner, the concentration of particulates in the buffy coat layer BC in Fig. 3 is reduced, whereby the white blood cell layer WBC, having a larger specific gravity than platelets, sediments in the outer side of the buffy coat layer BC. That is, the platelet layer PLT and the white blood cell layer WBC can further distinctly be separated.

Then after the circulation step performed for a certain period of time, an acceleration step (the fifth step) in the circulation/acceleration step illustrated in Fig. 10 starts. In the acceleration step, the rotational speed of the second blood pump P2 is controlled to gradually increase, thereby gradually increasing the flow rate of plasma. In the present example, the flow rate of plasma is raised from an initial flow rate of 100 ml/min to a rate where platelets start to flow out. This is performed in an acceleration period TG shown in Fig. 19. Fig. 4 illustrates the circulation step and the acceleration step represented by the circulation/acceleration step (S9, S12).

In the acceleration step, the platelets PLT receive ascending force and thereby flow out of the centrifuge bowl E1 through the outflow passage 19, as illustrated in Fig. 3. During this acceleration, the white blood cell layer WBC and the red blood cell layer RBC each having a large specific gravity, therefore receiving greater effect of centrifugal force, will not flow out from the outflow passage 19.

Fig. 20 illustrates changes in concentrations of platelets, white blood cells, and red blood cells flowing out. The horizontal axis represents the elapsed time during collecting platelets, and the vertical axis represents concentrations of blood cell components flowing out. First, platelets flow out (outflow period TA). In this period, the outflow rate of platelets gradually increases and, after peaking at the maximum flow rate, gradually decreases. Similarly, the outflow rate of white blood cells gradually increases and, after peaking at the maximum flow rate, gradually decreases.

Fig. 5 illustrates S9 and S12 in detail with a flowchart showing the operation of the blood component separation device.

The outflow period TA of platelets can be divided into three periods, that is, a low-concentration period TB, which comes first, where low-concentration platelet liquid flows out, a high-concentration period TC, following the TB period, where high-concentration platelet liquid flows out, and a low-concentration period TD, following the TC period, where low-concentration platelet liquid flows out again. Low-concentration platelet liquid is not necessary for obtaining high-concentration platelet liquid.

In the present example, in the acceleration step as illustrated in Fig. 10, when the turbidity sensor C2 detects platelets, that is, when it is determined that the present period is the TB period (S21: YES), the second open/close valve V2 is closed and the fifth open/close valve V5 is opened to store platelet liquid flowing out during the low-concentration period TB in Fig. 20 in the temporary storage bag Y2 (S22). In this state, since the whole blood also flows into the temporary storage bag Y2 and is stored therein, the low-concentration platelet liquid stored in the temporary storage bag Y2 is mixed with the whole blood. Also in this state, the first blood pump P1 is kept operating so that the whole blood drawn from the blood donor is continuously stored in the temporary storage bag Y2.

Note that, the temporary storage bag Y2 serves as a buffy coat bag as well as a whole blood bag.

When the turbidity sensor C2 detects that the concentration of platelet liquid is high, it is determined that the present period is the TC period (S23: YES), and the fifth open/close valve V5 is closed and the eighth open/close valve V8 is opened as illustrated in Fig. 11. In this manner, the high-concentration platelet liquid flowing out during the high-concentration period TC can be stored in the platelet intermediate bag Y3 (S24).

If the present cycle is not the last cycle (S7: NO), the first blood pump P1 is kept operating so that the whole blood drawn from the blood donor continuously flows through the sixth open/close valve V6 and is stored in the temporary storage bag Y2.

When a predetermined volume of high-concentration platelet liquid is stored in the platelet intermediate bag Y3, it is determined that the present period is the TD period (S25: YES), and the eighth open/close valve V8 is closed to block the low-concentration platelet liquid from flowing into the platelet intermediate bag Y3 and the fifth open/close valve V5 is opened, as illustrated in Fig. 12. In this manner, the low-concentration platelet liquid flowing out during the low-concentration period TD can be stored again in the temporary storage bag Y2 (S26).

If the present cycle is not the last cycle (S7: NO), the first blood pump P1 is kept operating so that the whole blood drawn from the blood donor continuously flows through the sixth open/close valve V6 and is stored in the temporary storage bag Y2.

The volume of high-concentration platelet liquid stored in the platelet intermediate bag Y3 can easily be adjusted by controlling the time period of opening the eighth open/close valve V8 based on the rate of platelet liquid flowing out of the centrifuge bowl E1.

Then upon finishing the collection of a predetermined volume of platelet liquid, in other words, when a predetermined time period has elapsed after opening the eighth open/close valve V8, it is determined that the TD period has past (S27: YES), or the outflow of platelets is stopped, and the step proceeds to the blood returning step illustrated in Fig. 13 (S10, S13).

In the blood returning step, the centrifuge bowl E1 stops rotation, the sixth open/close valve V6 and the fifth open/close valve V5 are closed, the first open/close valve V1 and the ninth open/close valve V9 are opened, and the first blood pump P1 is reversely rotated, whereby the returning of the blood remaining in the centrifuge bowl E1 to the blood donor starts. The first blood pump P1 is reversely operated at double the rotational speed of the normal rotation to shorten the time of blood returning. Further, as required, the second blood pump P2 is operated to return the excessive plasma stored in the plasma bag Y1.

When the blood returning finishes, and if the present cycle is the last cycle (S7: YES), the entire process is finished. When the finished cycle is not the last cycle (S7: NO), the centrifuge bowl E1 starts rotating as illustrated in Fig. 14, and the first blood pump P1 starts normal rotation again to perform blood drawing. The air inside the centrifuge bowl E1 (shown in dashed lines) is pushed by the plasma to flow out through the outflow passage 19 located in the inner periphery of the centrifuge bowl E1. The air then flows through the opened ninth open/close valve V9 and is stored in the air bag Y4. Simultaneously, by opening the seventh open/close valve V7 and operating the second blood pump P2, the blood stored in the temporary storage bag Y2 flows through the fourth open/close valve V4 into the centrifuge bowl E1 (S14). The third open/close valve V3 is closed to block the fluid from flowing into the plasma bag Y1.

Then when the turbidity sensor C2 detects that the fluid flowing in the tube has changed from air to plasma, the ninth open/close valve V9 is closed and the second open/close valve V2 is opened to store the plasma spilled out of the centrifuge bowl E1 in the plasma bag Y1, as illustrated in Fig. 15.

Then when it is confirmed that all the blood in the temporary storage bag Y2 has returned to the centrifuge bowl E1 and that a predetermined volume of plasma is stored in the plasma bag Y1 (S4: YES), the seventh open/close valve V7 is closed with the second blood pump P2 kept operating, and the third open/close valve V3 is opened to mix the plasma stored in the plasma bag Y1 with whole blood and to supply the mixture of the plasma and the whole blood to the centrifuge bowl E1, whereby the critical flow step of plasma is started, as illustrated in Fig. 16 (a state same as in Fig. 8). The step proceeds to the step illustrated in Fig. 9 (circulation step).

This cycle is repeated, typically three or four times, until a predetermined volume of platelets PLT is obtained. When the operation finishes with three cycles, for example, blood drawing is performed in parallel in a circulation period TF2 and an acceleration period TG2 in the second cycle to store whole blood in the temporary storage bag Y2. Then during blood drawing in the third cycle, the blood in the temporary storage bag Y2 is mixed with whole blood and supplied to the centrifuge bowl E1. Further, in a circulation period TF3 and an acceleration period TG3 in the third cycle, blood drawing is not performed. This is because there is no fourth cycle.

When the operation is to finish with three cycles, blood sampling finishes when the blood sampling needle 2 is removed from the blood donor after finishing the blood returning in the third cycle. As illustrated in Fig. 17, the high-concentration platelet liquid stored in the platelet intermediate bag Y3 is injected into the platelet bag Y5. Then as illustrated in Fig. 18, the platelet reserve liquid remaining in the platelet reserve liquid bottle is injected into the platelet bag Y5 through a bottle needle 10 coupled to the platelet reserve liquid bottle.

A method of correcting the HCT value (hematocrit value) measured with the blood cell counter 32 will now be described. In the present example, the correction of the HCT value measured with the blood cell counter 32 is performed automatically, and from the corrected HCT value, the estimated extracorporeal circulation volume, which is an estimated sampled blood volume (drawn volume of blood) per one cycle of the operation of collecting platelet liquid is calculated by a predetermined equation.

The HCT value measured with the blood cell counter 32 may have an error to deviate from the actual HCT value. This causes an error between the estimated extracorporeal circulation volume, which is the target sampled blood volume per one cycle, calculated in the controller 15 from the measured HCT value and the actual drawn volume of blood per one cycle measured with the processed blood volume measuring unit 34. This error may cause failure of the blood component separation circuit 1 to collect platelet liquid satisfying the target concentration (target number of platelets).

The present example is configured that the controller 15 calculates the true HCT correction value $\alpha$ from a data value A of the estimated extracorporeal circulation volume of a plurality of finished and immediately preceding operations of collecting platelet liquid and a data value B of the drawn volume of blood (sampled blood volume) measured with the processed blood volume measuring unit 34 in the first cycle of each of a plurality of finished and immediately preceding operations of collecting platelet liquid. The controller 15 corrects the HCT value measured with the blood cell counter 32 using the true HCT correction value $\alpha$ and then calculates the estimated extracorporeal circulation volume of the present collecting operation from the corrected HCT value. The controller 15 calculates the estimated extracorporeal circulation volume in each operation of collecting platelet liquid.

The true HCT correction value $\alpha$ is specifically calculated by the following equation. In the equation, $\alpha0$ is the present HCT correction value, A is the data value of the estimated extracorporeal circulation volume, and B is the data value of drawn volume of blood measured in the first cycle.

[Equation 1]

$$\alpha = \alpha0 \times (A/B)$$

The present HCT correction value (the correction value for the HCT value measured with the blood cell counter 32) is $\alpha0$.

The data value A of the estimated extracorporeal circulation volume is the average of data value of estimated extracorporeal circulation volumes in a plurality of finished and immediately preceding operations of collecting platelet liquid. The data value B of the drawn volume of blood measured in the first cycle is an average of data value of drawn volume of blood measured in the first cycle of each of a plurality of finished and immediately preceding operation of collecting platelet liquid. In this manner the data value A of the estimated extracorporeal circulation volume and the data value B of the drawn volume of blood measured in the first cycle are updated after finishing each operation of collecting platelet liquid as needed. Thus a true HCT correction value $\alpha$ is updated at every operation of collecting platelet liquid as needed. The estimated extracorporeal circulation volume is accurately calculated in accordance with the accuracy of measuring the HCT value of the blood cell counter 32.

The data value of each of a plurality of immediately preceding operations of collecting platelet liquid is preferably calculated without using the data value of the operation in which blood sampling is stopped before finishing the collection of platelet liquid (data value of an incomplete collecting operation).

Data values of a plurality of immediately preceding operations of collecting platelet liquid may be, for example, but not limited to, the data values of immediately preceding 50 times of operations of collecting platelet liquid. Alternatively, the data values may be those of immediately preceding operations of collecting platelet liquid less than 50 times (for example, data values of immediately preceding 10 to 20 times of operations of collecting platelet liquid).

For example, when the present HCT correction value $\alpha 0$ is 1.00, the data value A of the estimated extracorporeal circulation volume is 404 ml (see Fig. 21) and the data value B of the drawn volume of blood measured in the first cycle is 397 ml (see Fig. 21), the true HCT correction value $\alpha$ calculated by the Equation 1 is 1.02. Example data of the first cycle (indicated as "cycle 1" in the figure) and the second cycle (indicated as "cycle 2" in the figure) are illustrated in Fig. 21, where "n" is the number of collecting operations.

The controller 15 corrects the HCT value measured with the blood cell counter 32 using the true HCT correction value $\alpha$ to calculate the corrected HCT value. The estimated extracorporeal circulation volume of the present collecting operation is calculated from the corrected HCT value by using a predetermined equation.

In the present example, the controller 15 automatically corrects the HCT value measured with the blood cell counter 32 and calculates the estimated extracorporeal circulation volume by substituting the corrected HCT value in the predetermined equation.

The HCT correction value may be calculated for each centrifugal condition including the centrifugal speed, because the drawn volume of blood changes as the centrifugal speed (rotational speed of the centrifuge bowl E1) changes. The blood component separation device may be configured to turn on and off the function of correcting the HCT value.

As described above, the blood component separation device according to the first example includes the controller 15 which calculates the estimated extracorporeal circulation volume, or the target sampled blood volume per one cycle of the operation of collecting platelet liquid, from the HCT value calculated by correcting the HCT value measured with the blood cell counter 32. The controller 15 calculates the true HCT correction value $\alpha$, which is used for correcting the measured HCT value, from the data value A of the estimated extracorporeal circulation volume of a plurality of finished and immediately preceding operations of collecting platelet liquid and the data value B of the sampled blood volume measured in the first cycle of each of a plurality of finished and immediately preceding operations of collecting platelet liquid.

The true HCT correction value $\alpha$ is calculated from the data value of a plurality of immediately preceding operations of collecting platelet liquid. The measured HCT value is corrected using the calculated true HCT correction value $\alpha$ to calculate the estimated extracorporeal circulation volume of the present collecting operation. Since the measured HCT value is corrected in such a manner, the platelet liquid satisfying the target concentration can be collected even if the measuring accuracy of the blood cell counter 32 is low. The correction value for correcting the measured HCT value is updated upon finishing each collecting operation, so that the measured HCT value is suitably corrected along with the change in the measuring accuracy of the blood cell counter 32. For example, when the measuring accuracy of the blood cell counter 32 is calibrated, the measured HCT value is automatically corrected with the calibrated measuring accuracy of the blood cell counter 32 in the blood component separation device. This improves the accuracy of calculation of the estimated extracorporeal circulation volume. Thus the platelet liquid satisfying the target concentration can be collected regardless of the accuracy of measuring the HCT value of the blood cell counter 32.

The data value A of the estimated extracorporeal circulation volume of a plurality of immediately preceding operations of collecting platelet liquid and the data value B of the sampled blood volume measured in the first cycle of each of a plurality of immediately preceding operations of collecting platelet liquid are calculated without using the data value of the collecting operation in which blood sampling is stopped before finishing the operation of collecting platelet liquid. The true HCT correction value $\alpha$ is calculated using only the data value of the collecting operation succeeded in collecting platelet liquid. The platelet liquid satisfying the target concentration can thus surely be collected regardless of the accuracy of measuring the HCT value of the blood cell counter 32.

Furthermore, the blood component separation device according to the first example performs a) a centrifugal separation step of introducing whole blood drawn from a blood donor into the centrifuge bowl E1 to separate whole blood into a plurality of blood components, b) a circulation flow step of introducing plasma, among predetermined blood components separated in the centrifugal separation, into the centrifuge bowl E1 together with whole blood, c) a circulation/acceleration step, performed after a predetermined volume of plasma is separated in the circulation flow step, of stopping the supply of whole blood to the centrifugal separator to introduce only plasma into the centrifugal separator, further performing circulation for a predetermined period of time, and then increasing the circulation speed so that platelet liquid is separated in the centrifugal separator and collected, and d) a blood returning step, performed after collecting a predetermined volume of platelet liquid in the circulation/acceleration step, of returning blood components, which are not collected, to the blood donor. A cycle from the steps a) to d) is performed a plurality of times. In this manner, the platelet liquid can accurately be separated from other blood components.

Furthermore, the circulation/acceleration step performed in the blood component separation device according to the first example includes a first collecting step of transferring a portion of platelet liquid with low-concentration to a temporary storage container and a second collecting step of collecting a portion of platelet liquid with high-concentration. The platelet liquid with low-concentration transferred to the temporary storage container is introduced into the centrifuge bowl E1 together with the whole blood drawn in the following cycle. This process can be used for BC recycling (buffy coat recycling) to obtain platelet liquid with high-concentration, and thereby further larger volume of platelet liquid can be collected.

<Second Example>

Now, a second example will be described. The component similar to that of the first example is appended with the same reference sign and the description thereof will be omitted. Description will mainly be made for the difference between the second example and the first example. The major difference between the blood component separation device according to the second example and the blood component separation device of the first example is that the second example does not employ BC recycling. Fig. 22 illustrates a system configuration of a blood component separation device according to the second example. The major difference between the blood component separation circuit 30 according to the second example and the first example is that the second example does not include the temporary storage bag Y2.

The operation of the blood component separation device according to the second example will be described. Fig. 23 is a flow chart illustrating the operation of the blood component separation device. The operation and steps performed in the blood component separation device are illustrated in Figs. 24 to 26.

Similarly to the first example, the blood component separation device according to the second example first performs a priming step (S101). At the same time, as illustrated in Fig. 24, the drawing of whole blood and centrifugal separation start (S102: first blood sampling steep). Then the ninth open/close valve V9 is closed and the second open/close valve V2 is opened to store the plasma spilled out of the centrifuge bowl E1 in the plasma bag Y1.

When a certain volume of plasma is stored in the plasma bag Y1 (S103: YES), as illustrated in Fig. 25, the first open/close valve V1 is closed to temporarily stop the drawing of whole blood, and the third open/close valve V3 is opened to return the plasma into the centrifuge bowl E1 (S104: first circulation step).

Then the first open/close valve V1 is opened to restart drawing of whole blood to introduce the blood into the centrifuge bowl E1 (S105: second blood sampling step).

When the interface sensor C4 detects that the interface between the buffy coat layer BC and the red blood cell layer RBC in Fig. 3 has come to a predetermined position (S106: YES), similarly to the first circulation step, the first open/close valve V1 is closed to temporarily stop the drawing of whole blood, and the third open/close valve V3 is opened to return the plasma into the centrifuge bowl E1 (S107: second circulation step). The circulation speed is raised from 60 ml/min to as high as 170 to 200 ml/min.

The first open/close valve V1 is opened to restart drawing of whole blood. The sampled blood volume of whole blood is automatically calculated according to the HCT value so that the collection of platelet liquid can surely be performed (S108: third blood sampling step).

The first open/close valve V1 is then closed to temporarily stop the drawing of whole blood and to circulate the plasma to return to the centrifuge bowl E1, by gradually increasing the circulation speed (S109: acceleration step). The circulation speed is first increased from 60 ml/min to 150 ml/min, and eventually to 200 ml/min.

When the circulation speed exceeds 150 ml/min in the acceleration step, platelets starts to flow out. When the turbidity sensor C2 detects the outflow of platelets, as illustrated in Fig. 26, the eighth open/close valve V8 is opened to store platelet liquid in the platelet intermediate bag Y3 (S110: PC collecting step).

When the outflow of platelets has stopped, the step proceeds to the blood returning step (S111), similarly to the first example.

When the blood returning is finished, and when the finished cycle is the last cycle (S112: YES), the platelet liquid stored in the platelet intermediate bag Y3 is injected into the platelet bag Y5 through the white blood cell removal filter 11. Then the two tubes of the platelet bag are sealed. The platelet bag Y5 storing high-concentration platelet liquid is thus prepared. Now the entire step is finished. If the finished cycle is not the last cycle (S112: NO), the step proceeds to the first blood sampling step (S102).

Similarly to the example 1, the blood component separation device according to the second example described above is also configured that the controller 15 calculates the true HCT correction value α from the data value of a plurality of finished and immediately preceding operations of collecting platelet liquid so that the platelet liquid satisfying the target concentration can be collected regardless of the accuracy of measuring the HCT value of the blood cell counter 32.

The embodiments described above are merely exemplary representations and should not be construed to set any limit on the present invention. It goes without saying that various modifications and alterations can be made without departing from the spirit and scope of the present invention. In the embodiment described above, the temporary storage bag Y2 serves as a buffy coat bag as well as a whole blood bag, although the buffy coat bag may individually be provided in parallel with the whole blood bag.

Reference Signs List

| | |
|---|---|
| 1 | blood component separation circuit |
| 9 | sterilizing filter |
| 10 | bottle needle |
| 15 | controller |
| 30 | blood component separation circuit |
| 32 | blood cell counter |
| 34 | processed blood volume measuring unit |
| E1 | centrifuge bowl |
| Y1 | plasma bag (first container) |
| Y2 | temporary storage bag (second container) |
| Y3 | platelet intermediate bag (third container) |
| Y4 | air bag |
| Y5 | platelet bag |
| Y6 | air bag |
| C2 | turbidity sensor |
| C4 | interface sensor |
| P1 | first blood pump |
| P2 | second blood pump |
| P3 | third blood pump |
| V1 | first open/close valve |
| V2 | second open/close valve |
| V3 | third open/close valve |
| V4 | fourth open/close valve |
| V5 | fifth open/close valve |
| V6 | sixth open/close valve |
| V7 | seventh open/close valve |
| V8 | eighth open/close valve |
| V9 | ninth open/close valve |
| V10 | tenth open/close valve |
| T1 | to 21 tube |

## Claims

1. A blood component separation device (1, 30) including a centrifugal separator (E1) configured to separate a plurality of blood components from blood and a container for containing a predetermined blood component centrifugally separated, and configured to perform a plurality of steps of collecting the predetermined blood component which is separated, the blood component separation device (1,30) comprising a calculation unit (15) configured to calculate an estimated extracorporeal circulation volume as a target sampled blood volume per one cycle of an operation of collecting the predetermined blood component from a hematocrit value obtained by correcting a hematocrit value measured with a blood cell counter (32), the calculation unit (15) being configured to calculate a correction value used for the correction from a data value of the estimated extracorporeal circulation volume of a plurality of finished and immediately preceding collecting operations and a data value of sampled blood volume measured in a first cycle of each of the plurality of latest preceding collecting operations.

2. The blood component separation device (1, 30) according to claim 1, wherein a data value of the estimated extracorporeal circulation volume and a data value of sampled blood volume measured in the first cycle are calculated without using a data value of a collecting operation in which blood sampling is stopped before finishing the collecting operation.

3. The blood component separation device (1, 30) according to claim 1 or 2, said blood component separation device being configured to perform the following steps:

    a) a centrifugal separation step of introducing whole blood drawn from a blood donor into a centrifugal separator (E1) to separate whole blood into a plurality of blood components;
    b) a circulation flow step of introducing a first blood component, among predetermined blood components separated in the centrifugal separation, into the centrifugal separator (E1) together with whole blood;
    c) a circulation/acceleration step, performed after a predetermined volume of the first blood component is separated in the circulation flow step, of stopping supply of whole blood to the centrifugal separator (E1) to introduce only the first blood component into the centrifugal separator, further performing circulation for a predetermined period of time, and then increasing a circulation speed so that a second blood component is separated in the centrifugal separator (E1) and collected; and
    d) a blood returning step, performed after collecting a predetermined volume of the second blood component in the circulation/acceleration step, of returning blood components, which are not collected, to the blood donor,

the steps a) to d) being performed as one cycle a plurality of times.

**4.** The blood component separation device (1, 30) according to claim 3, wherein
the circulation/acceleration step includes a first collecting step of transferring a portion of the second blood component with low-concentration among the second blood components to a temporary storage container, and a second collecting step of collecting a portion of the second blood component with high-concentration among the second blood components, and
the second blood component with low-concentration transferred to the temporary storage container is introduced into the centrifugal separator (E1) together with whole blood drawn in a following cycle.

**5.** The blood component separation device (1, 30) according to claim 1, wherein
the predetermined blood component is platelet liquid.

## Patentansprüche

**1.** Blutbestandteiltrennvorrichtung (1, 30) mit einem Zentrifugalabscheider (E1), der konfiguriert ist, um mehrere Blutbestandteile aus Blut abzutrennen, und mit einem Behälter zum Enthalten eines vorher festgelegten Blutbestandteils, der zentrifugal abgetrennt wurde, und die konfiguriert ist, um mehrere Schritte des Sammelns des vorher festgelegten Blutbestandteils, der abgetrennt ist, wobei die Blutbestandteiltrennvorrichtung (1, 30) eine Recheneinheit (15) aufweist, die konfiguriert ist, um ein geschätztes extrakorporales Zirkulationsvolumen als ein gesammeltes Zielblutvolumen pro Zyklus eines Vorgangs des Sammelns des vorher festgelegten Blutbestandteils aus einem Hämatokritwert zu berechnen, der durch Korrigieren eines mit einem Blutzellzähler (32) gemessenen Hämatokritwertes erhalten wurde, wobei die Recheneinheit (15) konfiguriert ist, um einen Korrekturwert, der für die Korrektur verwendet wird, aus einem Datenwert des geschätzten extrakorporalen Zirkulationsvolumens einer Mehrzahl von fertiggestellten und unmittelbar vorangehenden Sammelvorgängen und aus einem Datenwert des gesammelten Blutvolumens zu berechnen, das in einem ersten Zyklus von jedem der Mehrzahl der letzten vorangehenden Sammelvorgänge gemessen wurde.

**2.** Blutbestandteiltrennvorrichtung (1, 30) nach Anspruch 1, wobei
ein Datenwert des geschätzten extrakorporalen Zirkulationsvolumens und ein Datenwert des gesammelten Blutvolumens, das in dem ersten Zyklus gemessen wurde, ohne Verwendung eines Datenwerts eines Sammelvorgangs berechnet werden, bei dem die Blutentnahme vor dem Beenden des Sammelvorgangs gestoppt wird.

**3.** Blutbestandteiltrennvorrichtung (1, 30) nach Anspruch 1 oder 2, wobei die Blutbestandteiltrennvorrichtung konfiguriert ist, um einen der folgenden Schritte durchzuführen:

a) einen zentrifugalen Trennungsschritt zum Einleiten von Vollblut, das einem Spender abgenommen wurde, in den Zentrifugalabscheider (E1), um das Vollblut in mehrere Blutbestandteile zu trennen;
b) einen Zirkulationsströmungsschritt zum Einleiten eines ersten Blutbestandteils, unter den vorher festgelegten, in der zentrifugalen Abtrennung abgetrennten Blutbestandteilen, in den Zentrifugalabscheider (E1) zusammen mit Vollblut;
c) einen Zirkulations- / Beschleunigungsschritt, der durchgeführt wird, nachdem ein vorbestimmtes Volumen des ersten Blutbestandteils in dem Zirkulationsströmungsschritt abgetrennt worden ist, zum Stoppen der Zufuhr von Vollblut zu dem Zentrifugalabscheider (E1), um nur den ersten Blutbestandteil in den Zentrifugalabscheider einzuleiten, wobei weiterhin eine Zirkulation für eine vorgegebene Zeitspanne durchgeführt wird, und um anschließend eine Zirkulationsgeschwindigkeit zu erhöhen, sodass ein zweiter Blutbestandteil in dem Zentrifugalabscheider (E1) abgetrennt und gesammelt wird; und
d) einen Blutrückführungsschritt, der nach dem Sammeln eines vorher festgelegten Volumens des zweiten Blutbestandteils in dem Zirkulations- / Beschleunigungsschritt, zur Rückführung von Blutbestandteilen, die nicht gesammelt wurden, an den Spender durchgeführt wird,

wobei die Schritte a) bis d) mehrmals als ein Zyklus durchgeführt werden.

**4.** Blutbestandteiltrennvorrichtung (1, 30) nach Anspruch 3, wobei
der Zirkulations- / Beschleunigungsschritt einen ersten Sammelschritt des Übertragens eines Teils des zweiten Blutbestandteils mit einer niedrigen Konzentration zwischen den zweiten Blutbestandteilen zu einem Zwischenspeicherbehälter, und einen zweiten Sammelschritt zum Sammeln eines Teils der zweiten Blutbestandteil mit hohen -Konzentration unter den zweiten Blutbestandteilen umfasst, und der zweite Blutbestandteil mit geringer Konzentration, der in den Zwischenspeicherbehälter überführt wurde, zusammen mit dem in einem folgenden Zyklus abgenommenen Vollblut in den Zentrifugalabscheider (E1) eingeführt wird.

**5.** Blutbestandteiltrennvorrichtung (1, 30) nach Anspruch 1, wobei der vorher festgelegte Blutbestandteil Plättchenflüssigkeit ist.

**Revendications**

**1.** Dispositif de séparation des composants du sang (1, 30) comprenant un séparateur centrifuge (E1) conçu pour séparer du sang une pluralité de composants sanguins et un contenant destiné à contenir un composant sanguin prédéterminé séparé par centrifugation, et conçu pour effectuer une pluralité d'étapes consistant à collecter le composant sanguin prédéterminé qui est séparé, le dispositif de séparation des composants du sang (1, 30) comprenant une unité de calcul (15) conçue pour calculer un volume de circulation extracorporelle estimé en tant que volume de sang prélevé cible par cycle d'opération de collecte du composant sanguin prédéterminé à partir d'une valeur d'hématocrite obtenue en corrigeant une valeur d'hématocrite mesurée avec un compteur de cellules sanguines (32), l'unité de calcul (15) étant conçue pour calculer une valeur de correction utilisée pour la correction à partie d'une valeur de données du volume de circulation extracorporelle estimé d'une pluralité d'opérations de collecte finies et immédiatement précédentes et d'une valeur de données de volume de sang prélevé mesuré dans un premier cycle de chacune de la pluralité des dernières opérations de collecte précédentes.

**2.** Dispositif de séparation des composants du sang (1, 30) selon la revendication 1, dans lequel une valeur de données du volume de circulation extracorporelle estimé et une valeur de données du volume de sang prélevé mesurées dans le premier cycle sont calculées sans utiliser de valeur de données d'une opération de collecte dans laquelle le prélèvement de sang est arrêté avant de terminer l'opération de collecte.

**3.** Dispositif de séparation des composants du sang (1, 30) selon la revendication 1 ou 2, ledit dispositif de séparation des composants du sang étant conçu pour effectuer les étapes suivantes :

(a) une étape de séparation centrifuge consistant à introduire du sang complet prélevé chez un donneur dans un séparateur centrifuge (E1) afin de séparer le sang complet en une pluralité de composants sanguins ;
(b) une étape de circulation consistant à introduire un premier composant sanguin, parmi des composants sanguins prédéterminés séparés lors de la séparation centrifuge, dans le séparateur centrifuge (E1) en même que le sang complet ;
(c) une étape d'accélération/circulation, effectuée après qu'un volume prédéterminé du premier composant sanguin est séparé dans l'étape de circulation, consistant à arrêter l'introduction de sang complet dans le séparateur centrifuge (E1) pour n'introduire que le premier composant sanguin dans le séparateur centrifuge, en effectuant en outre une circulation pendant une période de temps prédéterminée, et en augmentant ensuite une vitesse de circulation de sorte qu'un second composant sanguin est séparé dans le séparateur centrifuge (E1) et collecté ; et
(d) une étape de retour de sang, effectuée après la collecte d'un volume prédéterminé du second composant sanguin dans l'étape d'accélération/circulation, consistant à renvoyer les composants sanguins, qui ne sont pas collectés, au donneur,

les étapes (a) à (d) étant effectuées plusieurs fois en un seul cycle.

**4.** Dispositif de séparation des composants du sang (1, 30) selon la revendication 3, dans lequel l'étape d'accélération/circulation comprend une première étape de collecte destinée à transférer une partie du second composant sanguin de faible concentration parmi les seconds composants sanguins dans un contenant de stockage temporaire, et une seconde étape de collecte destinée à collecter une partie du second composant sanguin de concentration élevée parmi les seconds composants sanguins, et le second composant sanguin de faible concentration transféré dans le contenant de stockage temporaire est introduit dans le séparateur centrifuge (E1) en même temps que le sang complet prélevé dans un cycle suivant.

**5.** Dispositif de séparation des composants du sang (1, 30) selon la revendication 1, dans lequel le composant sanguin prédéterminé est un liquide de plaquettes.

FIG. 1

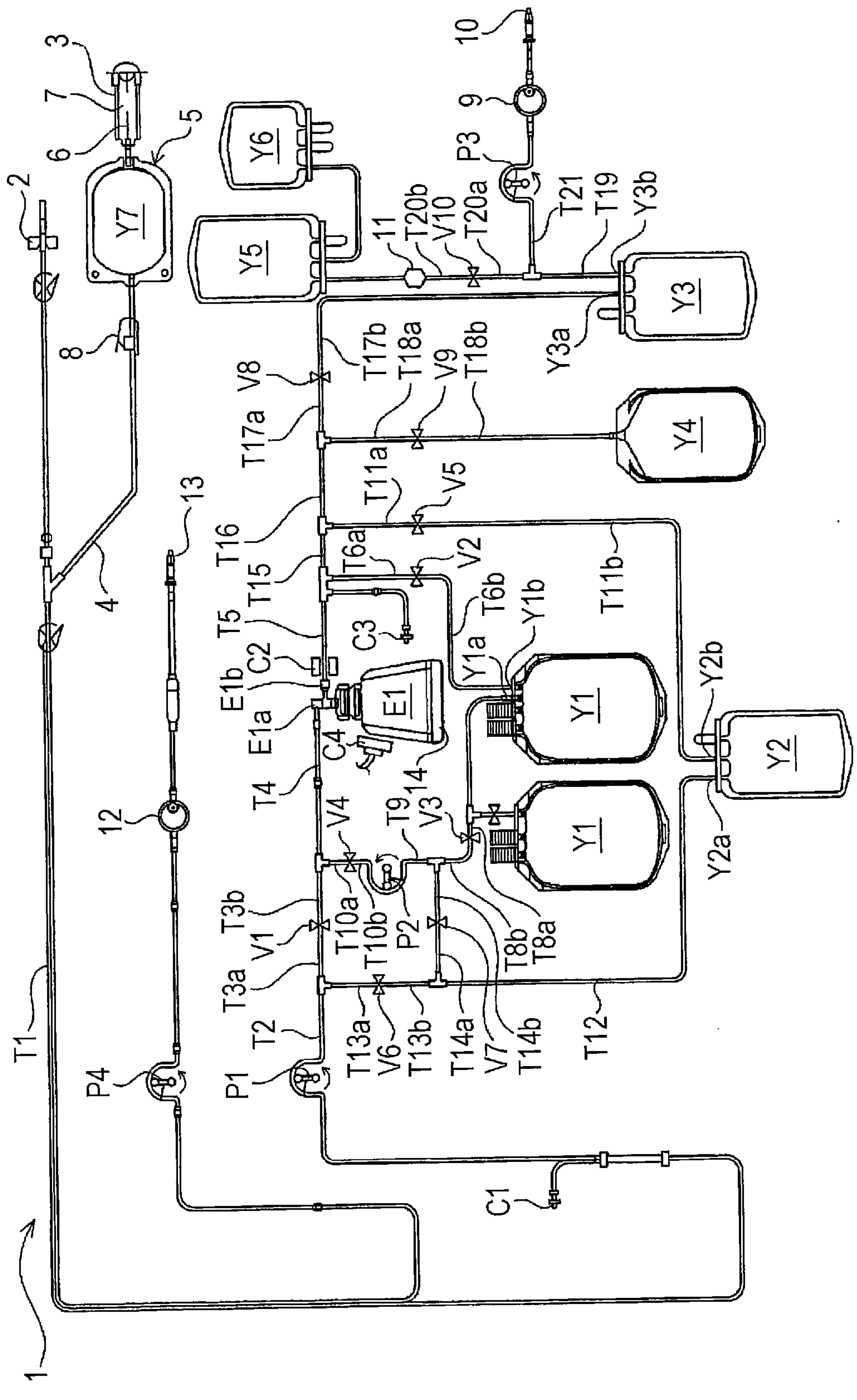
1
2
3
4
5
6
7
8
9
10
11
12
13
14
Y1
Y2
Y3
Y4
Y5
Y6
Y7
E1
E1a
E1b
C1
C2
C3
C4
P1
P2
P3
P4
V1
V2
V3
V4
V5
V6
V7
V8
V9
V10
T1
T2
T3a
T3b
T4
T5
T6a
T6b
T8a
T8b
T9
T10a
T10b
T11a
T11b
T12
T13a
T13b
T14a
T14b
T15
T16
T17a
T17b
T18a
T18b
T19
T20a
T20b
T21
Y1a
Y1b
Y2a
Y2b
Y3a
Y3b

FIG. 2

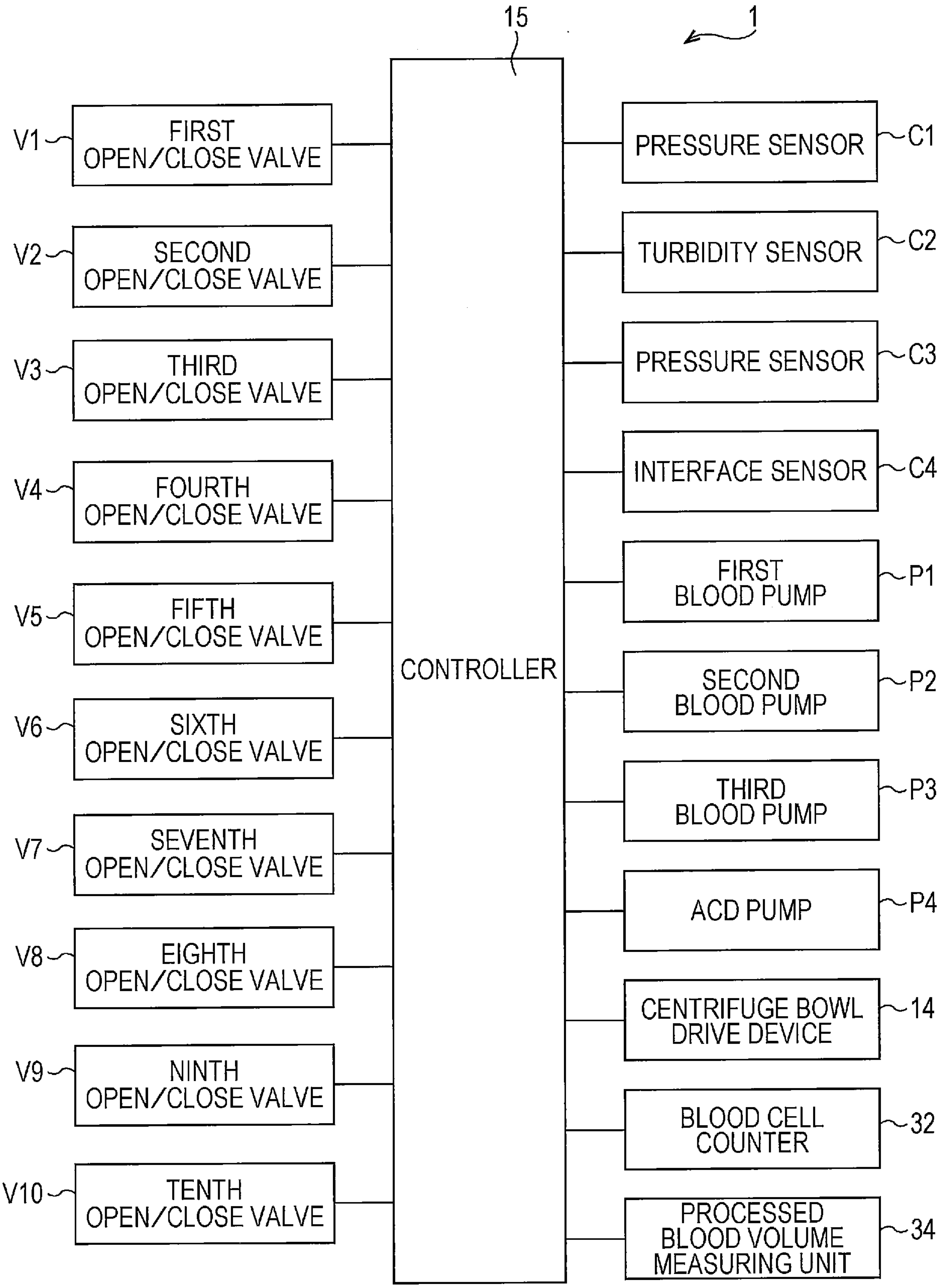
15
1
CONTROLLER
V1 FIRST OPEN/CLOSE VALVE
V2 SECOND OPEN/CLOSE VALVE
V3 THIRD OPEN/CLOSE VALVE
V4 FOURTH OPEN/CLOSE VALVE
V5 FIFTH OPEN/CLOSE VALVE
V6 SIXTH OPEN/CLOSE VALVE
V7 SEVENTH OPEN/CLOSE VALVE
V8 EIGHTH OPEN/CLOSE VALVE
V9 NINTH OPEN/CLOSE VALVE
V10 TENTH OPEN/CLOSE VALVE
PRESSURE SENSOR C1
TURBIDITY SENSOR C2
PRESSURE SENSOR C3
INTERFACE SENSOR C4
FIRST BLOOD PUMP P1
SECOND BLOOD PUMP P2
THIRD BLOOD PUMP P3
ACD PUMP P4
CENTRIFUGE BOWL DRIVE DEVICE 14
BLOOD CELL COUNTER 32
PROCESSED BLOOD VOLUME MEASURING UNIT 34

FIG. 3

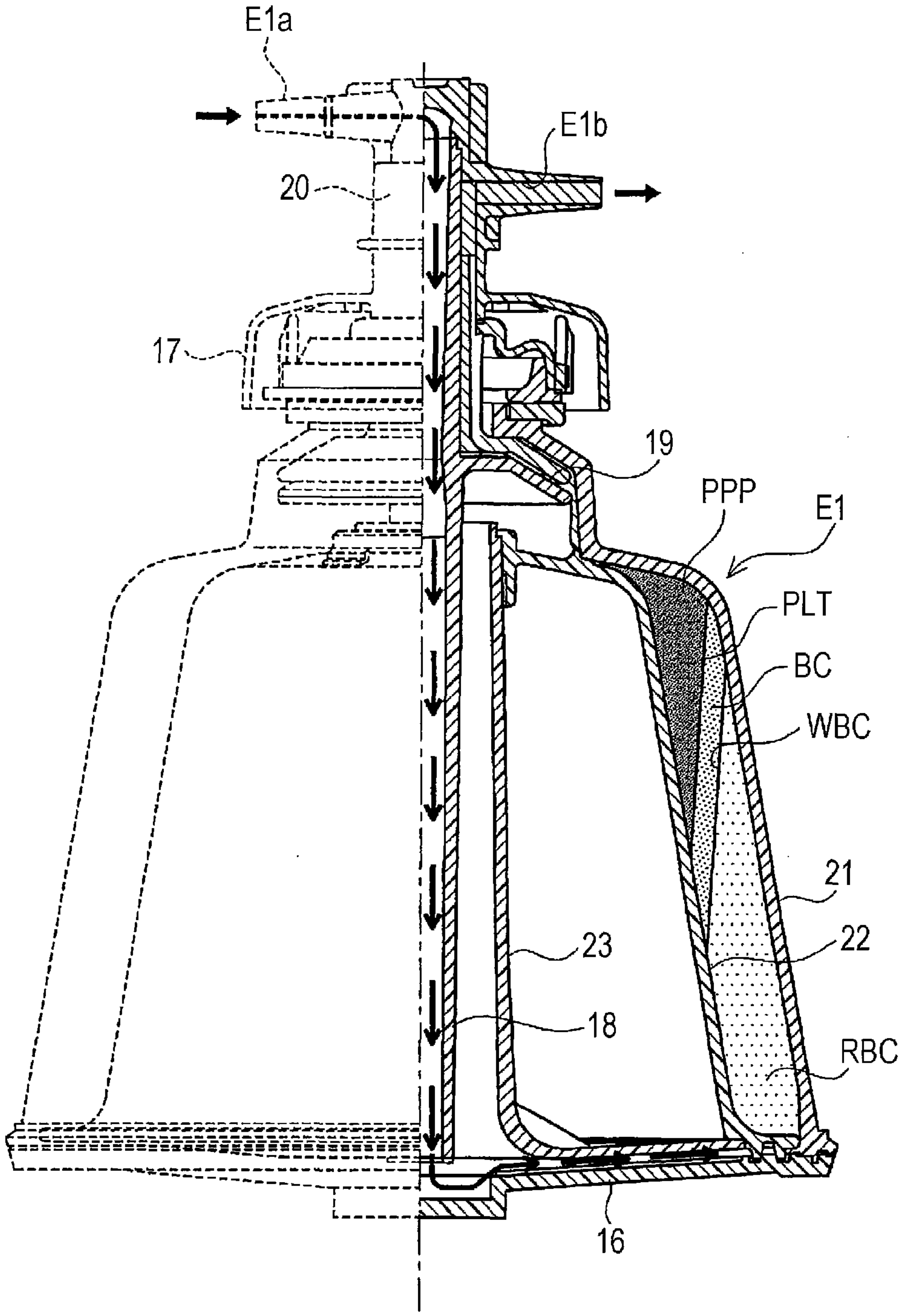

FIG. 4

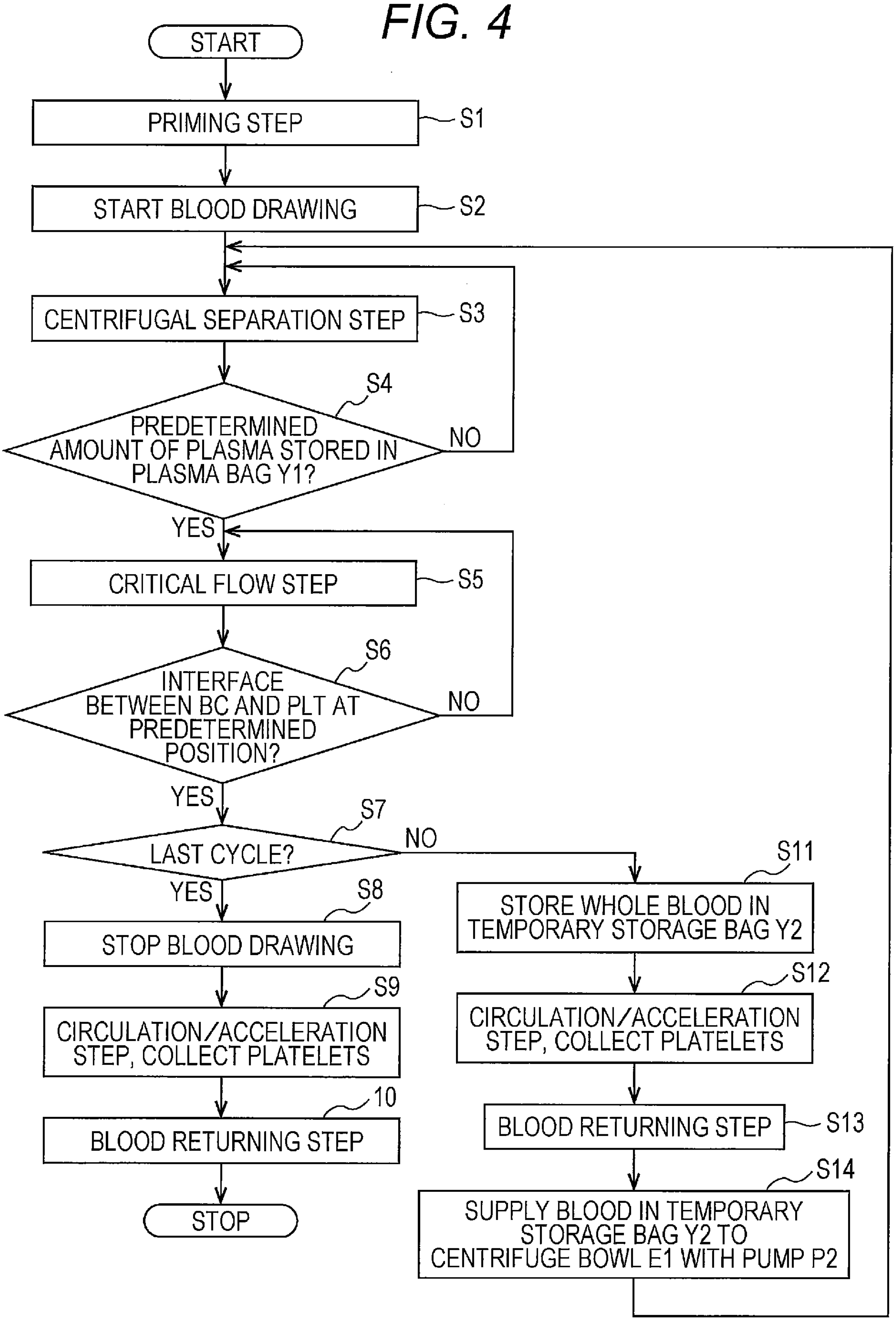
START
PRIMING STEP
S1
START BLOOD DRAWING
S2
CENTRIFUGAL SEPARATION STEP
S3
S4
PREDETERMINED AMOUNT OF PLASMA STORED IN PLASMA BAG Y1?
NO
YES
CRITICAL FLOW STEP
S5
S6
INTERFACE BETWEEN BC AND PLT AT PREDETERMINED POSITION?
NO
YES
S7
LAST CYCLE?
NO
YES
S8
STOP BLOOD DRAWING
S9
CIRCULATION/ACCELERATION STEP, COLLECT PLATELETS
10
BLOOD RETURNING STEP
STOP
S11
STORE WHOLE BLOOD IN TEMPORARY STORAGE BAG Y2
S12
CIRCULATION/ACCELERATION STEP, COLLECT PLATELETS
BLOOD RETURNING STEP
S13
S14
SUPPLY BLOOD IN TEMPORARY STORAGE BAG Y2 TO CENTRIFUGE BOWL E1 WITH PUMP P2

FIG. 5

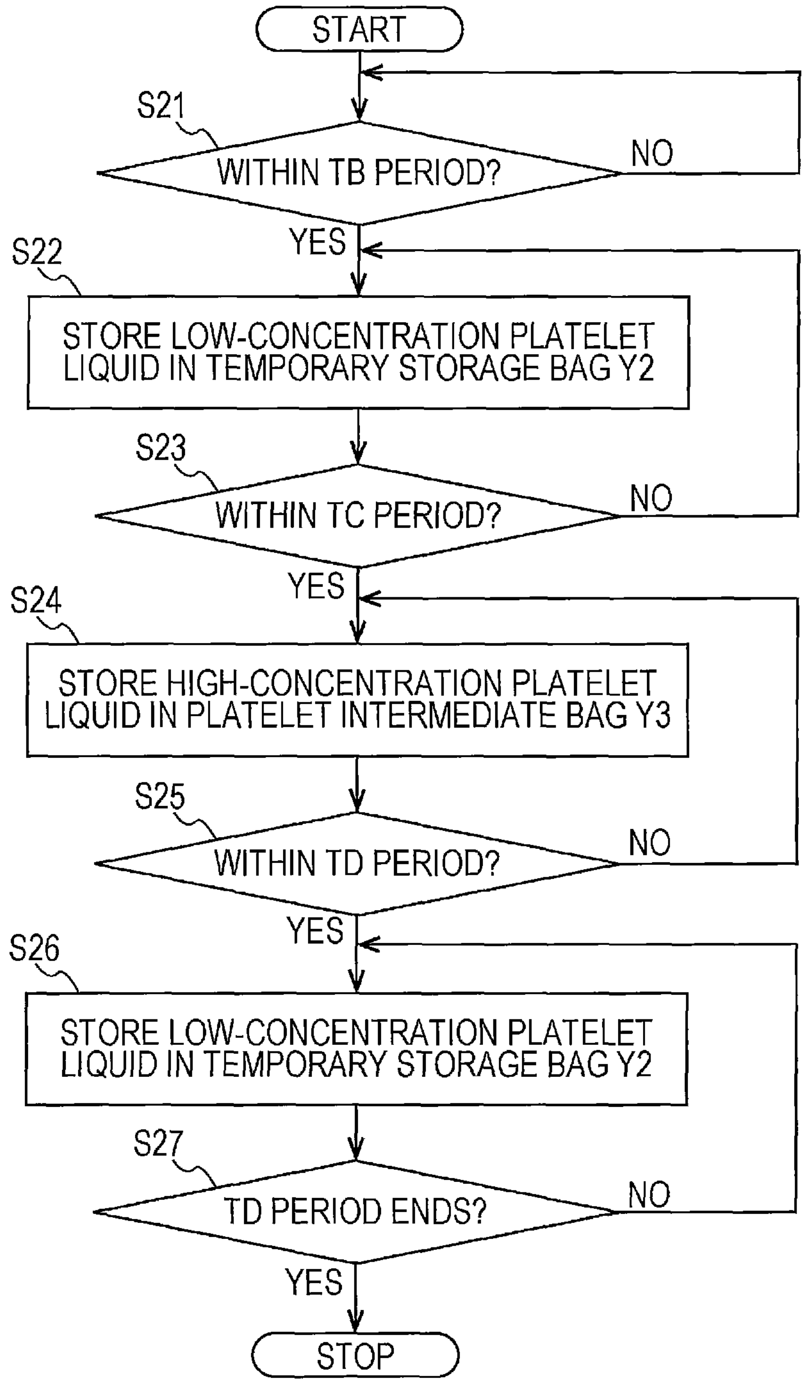
START
S21
WITHIN TB PERIOD?
NO
YES
S22
STORE LOW-CONCENTRATION PLATELET LIQUID IN TEMPORARY STORAGE BAG Y2
S23
WITHIN TC PERIOD?
NO
YES
S24
STORE HIGH-CONCENTRATION PLATELET LIQUID IN PLATELET INTERMEDIATE BAG Y3
S25
WITHIN TD PERIOD?
NO
YES
S26
STORE LOW-CONCENTRATION PLATELET LIQUID IN TEMPORARY STORAGE BAG Y2
S27
TD PERIOD ENDS?
NO
YES
STOP

FIG. 6

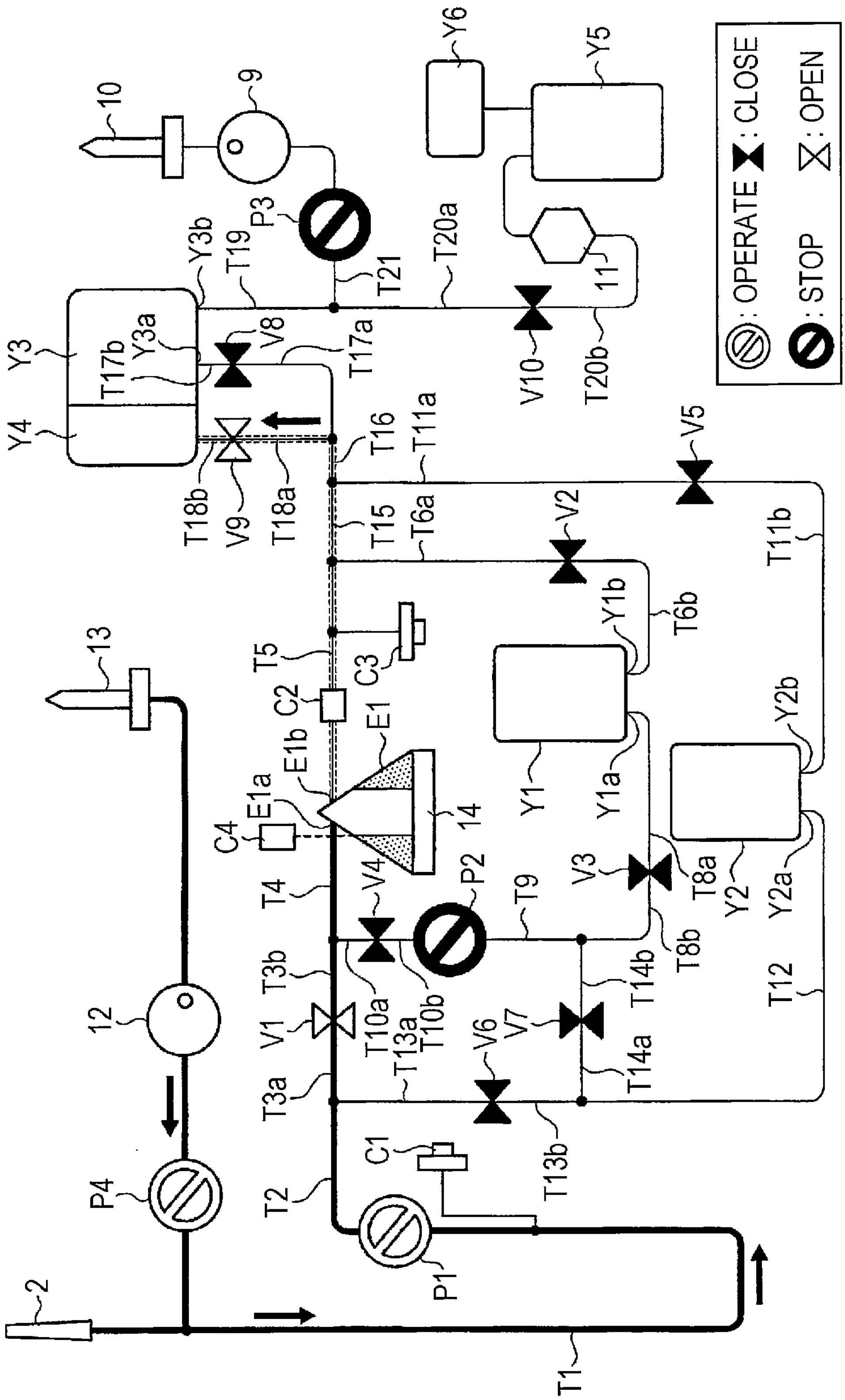

FIG. 7

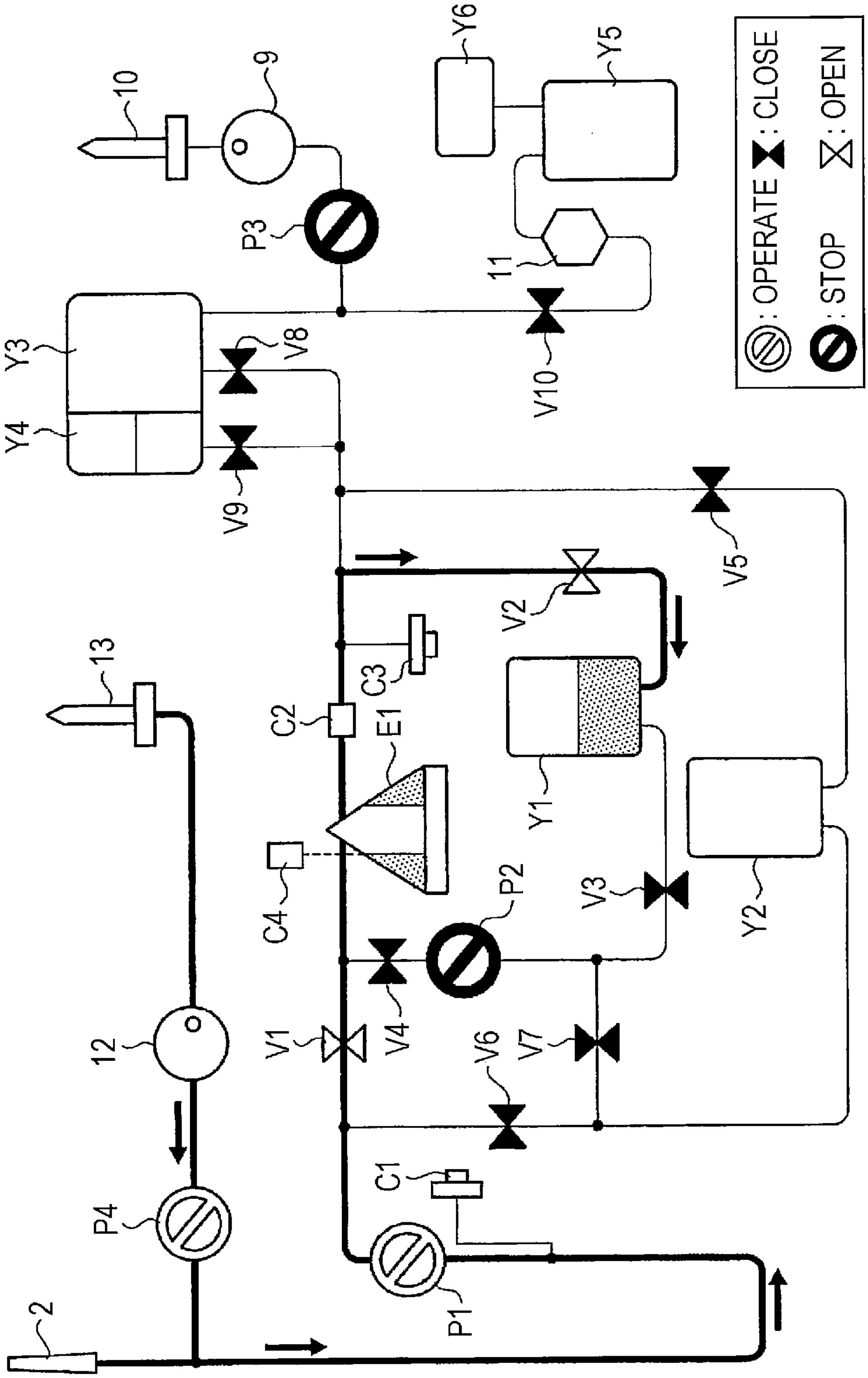
: OPERATE
: CLOSE
: STOP
: OPEN
2
P1
P2
P3
P4
C1
C2
C3
C4
E1
V1
V2
V3
V4
V5
V6
V7
V8
V9
V10
Y1
Y2
Y3
Y4
Y5
Y6
9
10
11
12
13

FIG. 8

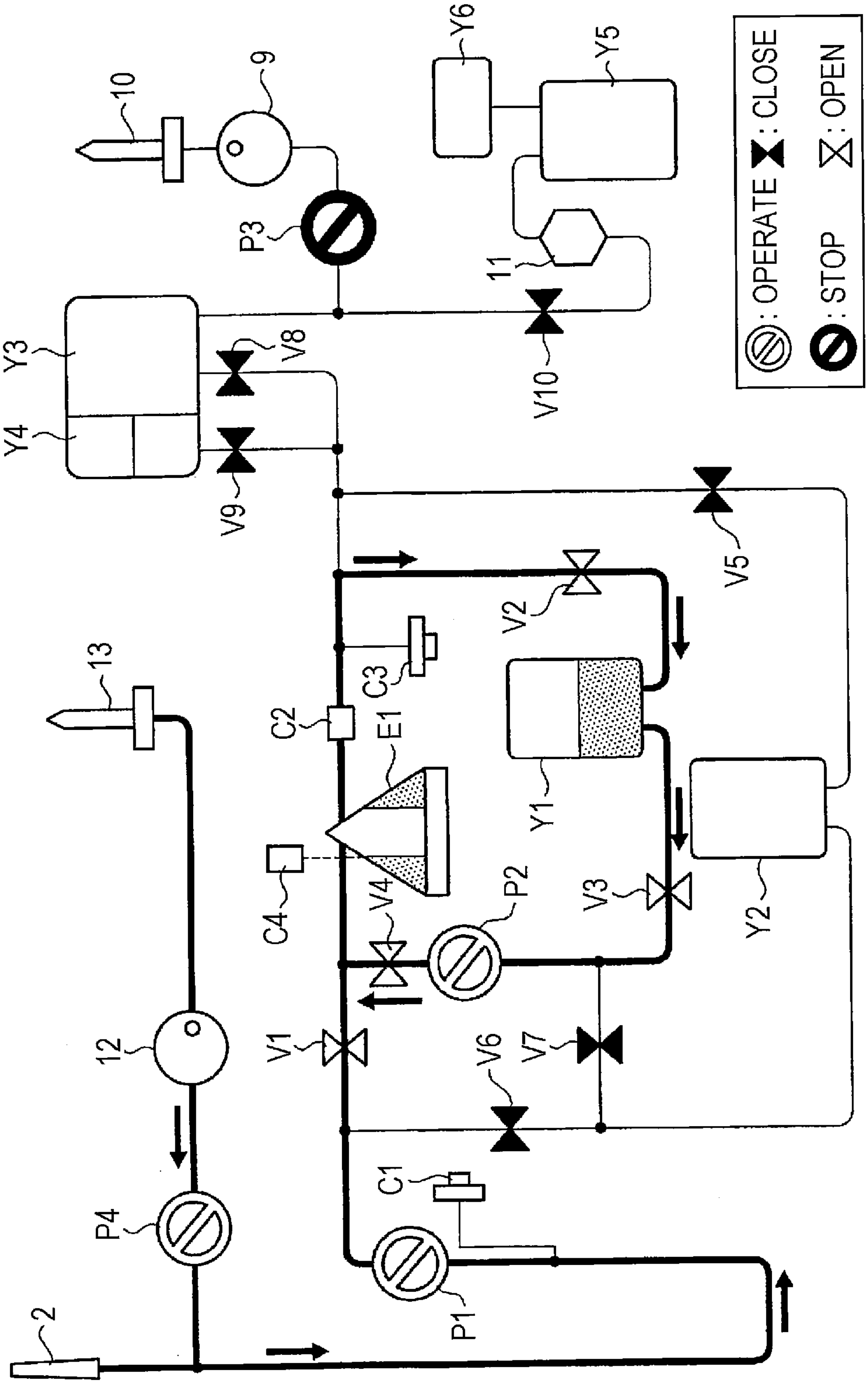

2
P1
P4
12
13
C1
V1
V6
V7
V4
C4
P2
V3
E1
C2
C3
Y1
Y2
V2
V5
V9
V8
Y4
Y3
V10
11
P3
9
10
Y6
Y5
: OPERATE
: CLOSE
: STOP
: OPEN

FIG. 9

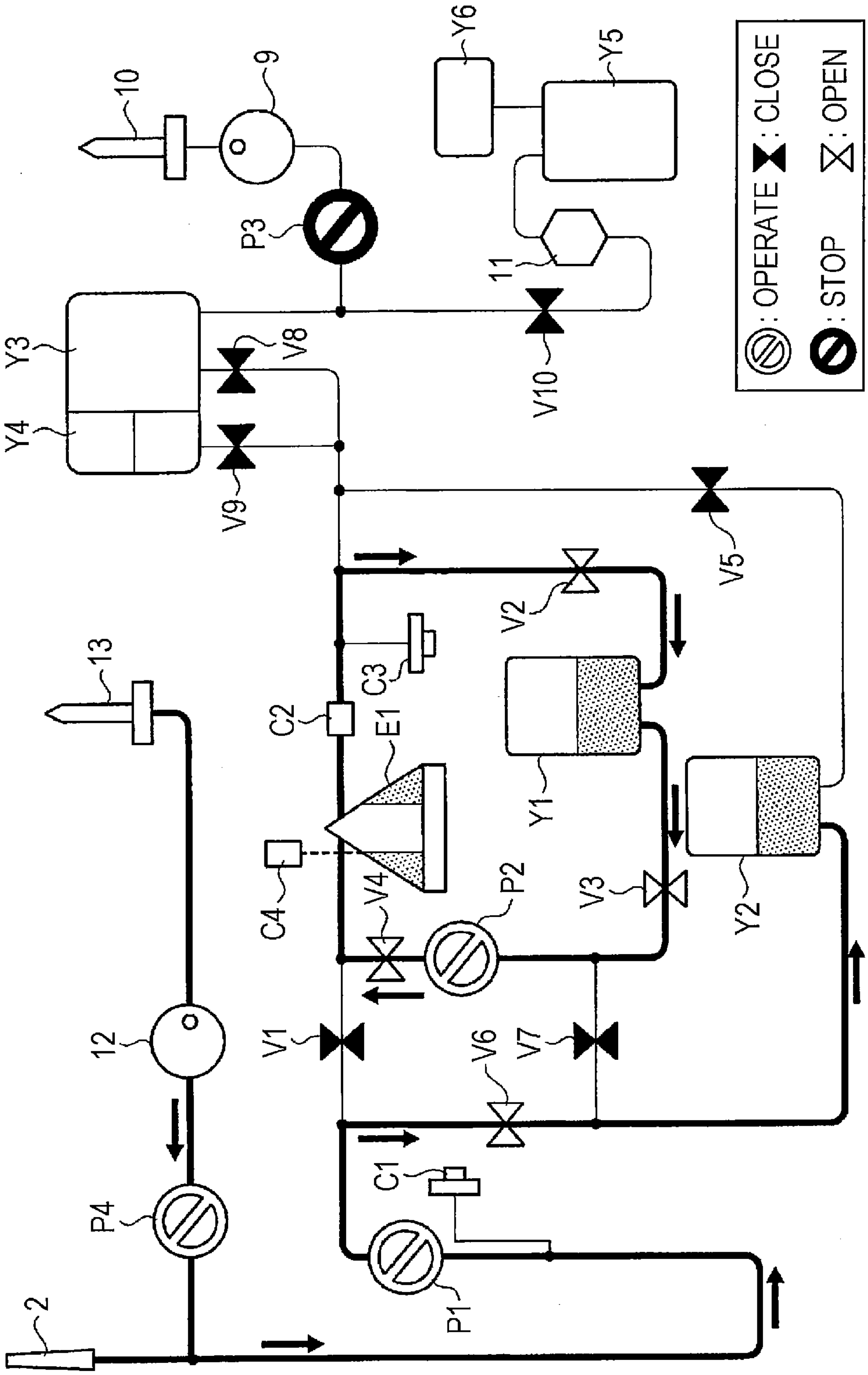
2
P4
12
13
P1
C1
V6
V7
V1
V4
P2
C4
E1
C2
C3
V3
Y1
Y2
V2
V5
V9
V8
Y4
Y3
V10
11
P3
9
10
Y5
Y6
: OPERATE
: CLOSE
: STOP
: OPEN

FIG. 10

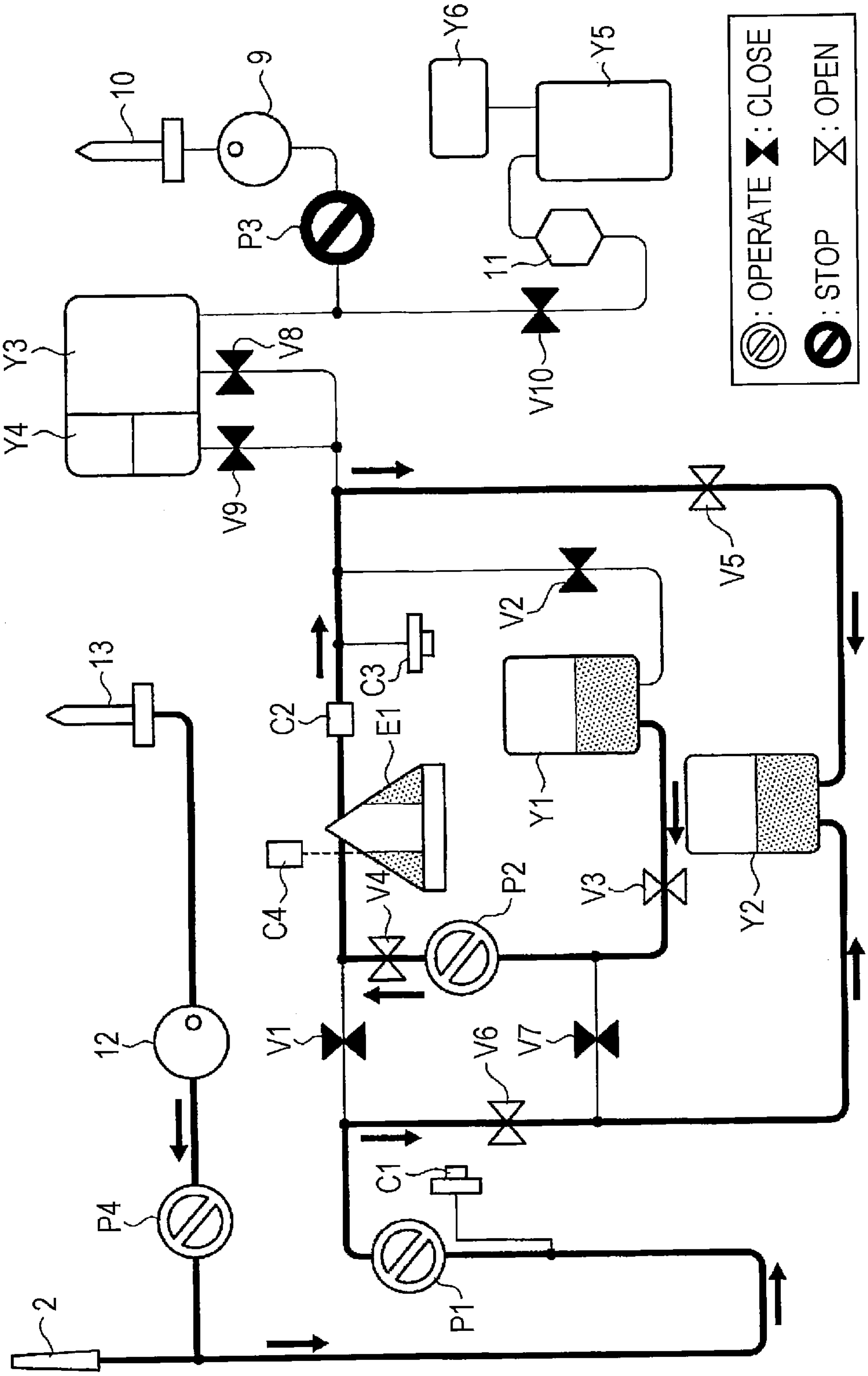
2
P1
P2
P3
P4
C1
C2
C3
C4
E1
V1
V2
V3
V4
V5
V6
V7
V8
V9
V10
Y1
Y2
Y3
Y4
Y5
Y6
9
10
11
12
13
: OPERATE
: CLOSE
: STOP
: OPEN

FIG. 11

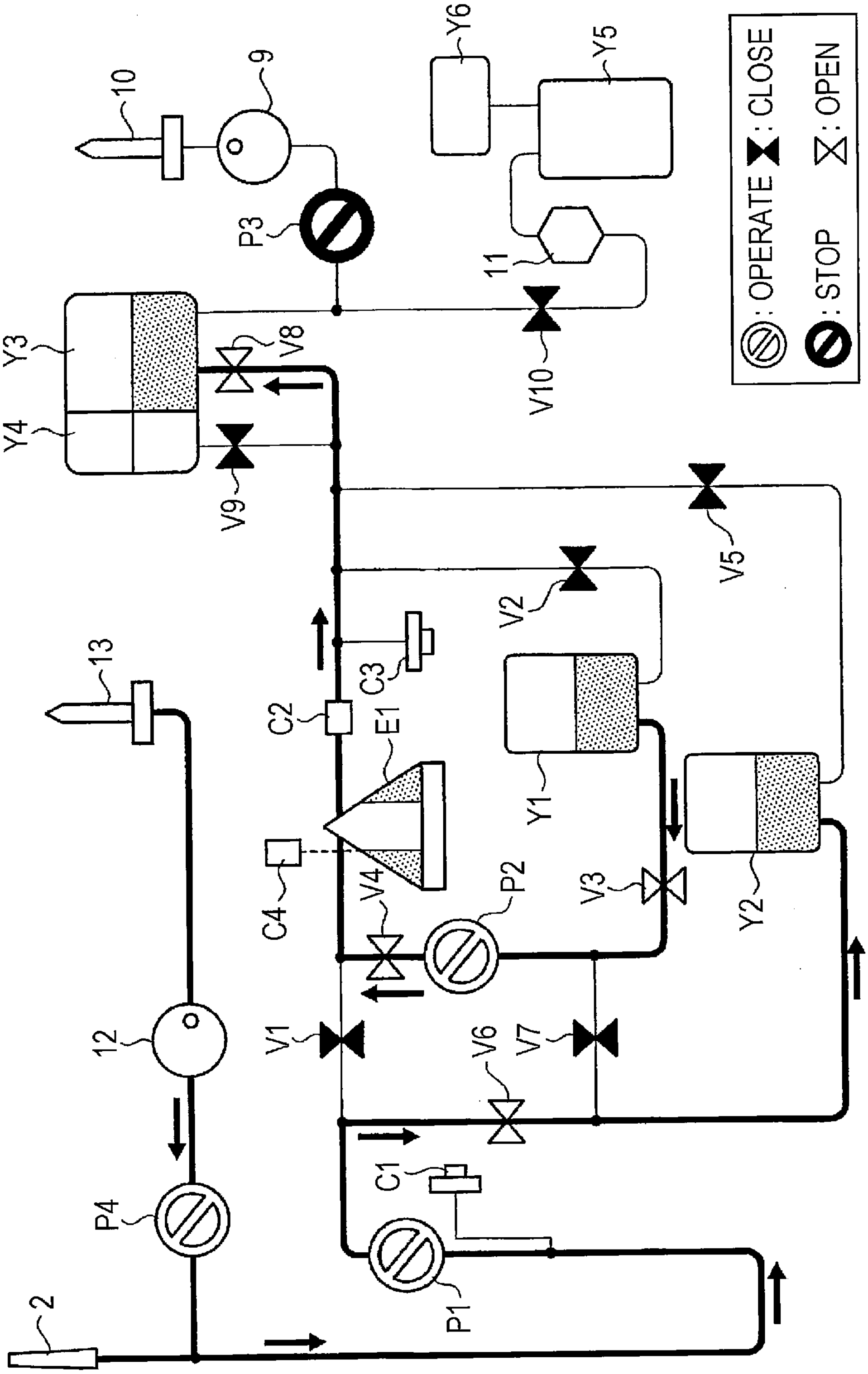
2
P4
12
13
10
9
P3
Y3
Y4
V8
V9
V10
11
Y5
Y6
P1
C1
V6
V7
V1
V4
P2
C4
E1
C2
C3
V3
Y1
Y2
V2
V5
: OPERATE
: CLOSE
: STOP
: OPEN

FIG. 12

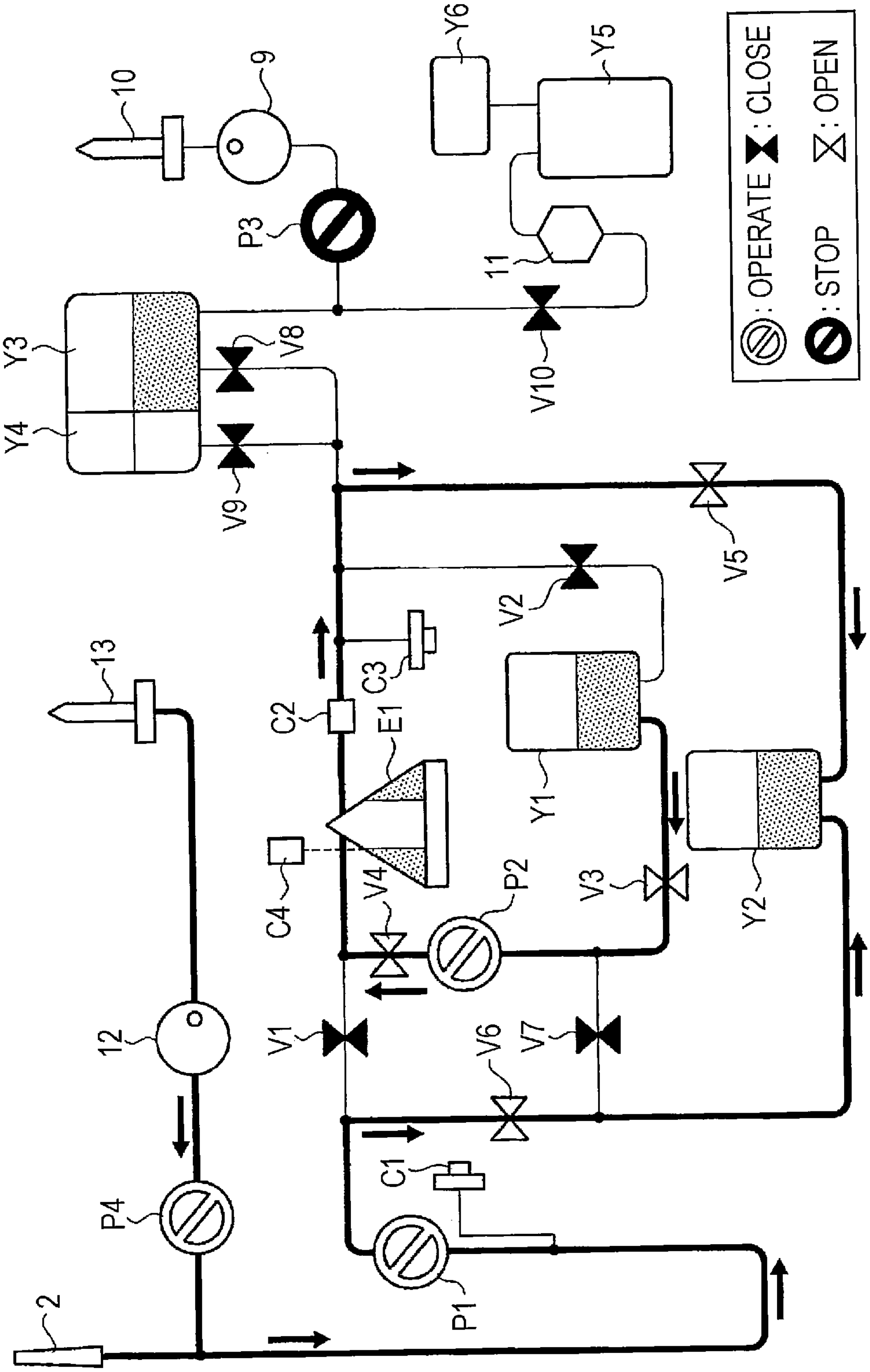
10
9
Y6
Y5
P3
11
Y3
Y4
V8
V9
V10
V5
V2
C3
C2
E1
Y1
13
C4
V4
P2
V3
Y2
12
V1
V6
V7
C1
P4
P1
2
: OPERATE
: CLOSE
: STOP
: OPEN

FIG. 13

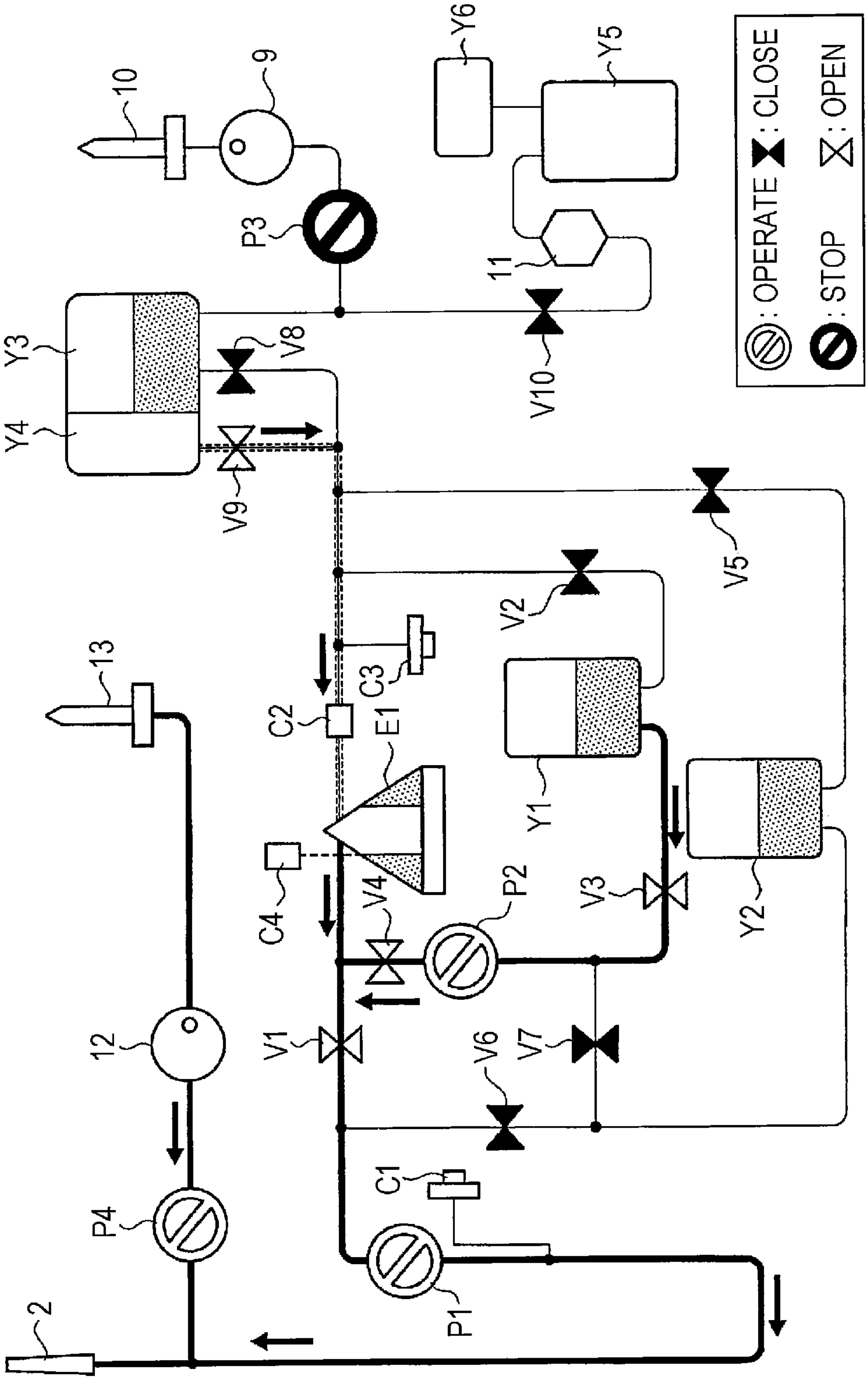
2
P1
P2
P3
P4
C1
C2
C3
C4
E1
V1
V2
V3
V4
V5
V6
V7
V8
V9
V10
Y1
Y2
Y3
Y4
Y5
Y6
9
10
11
12
13
: OPERATE
: CLOSE
: STOP
: OPEN

FIG. 14

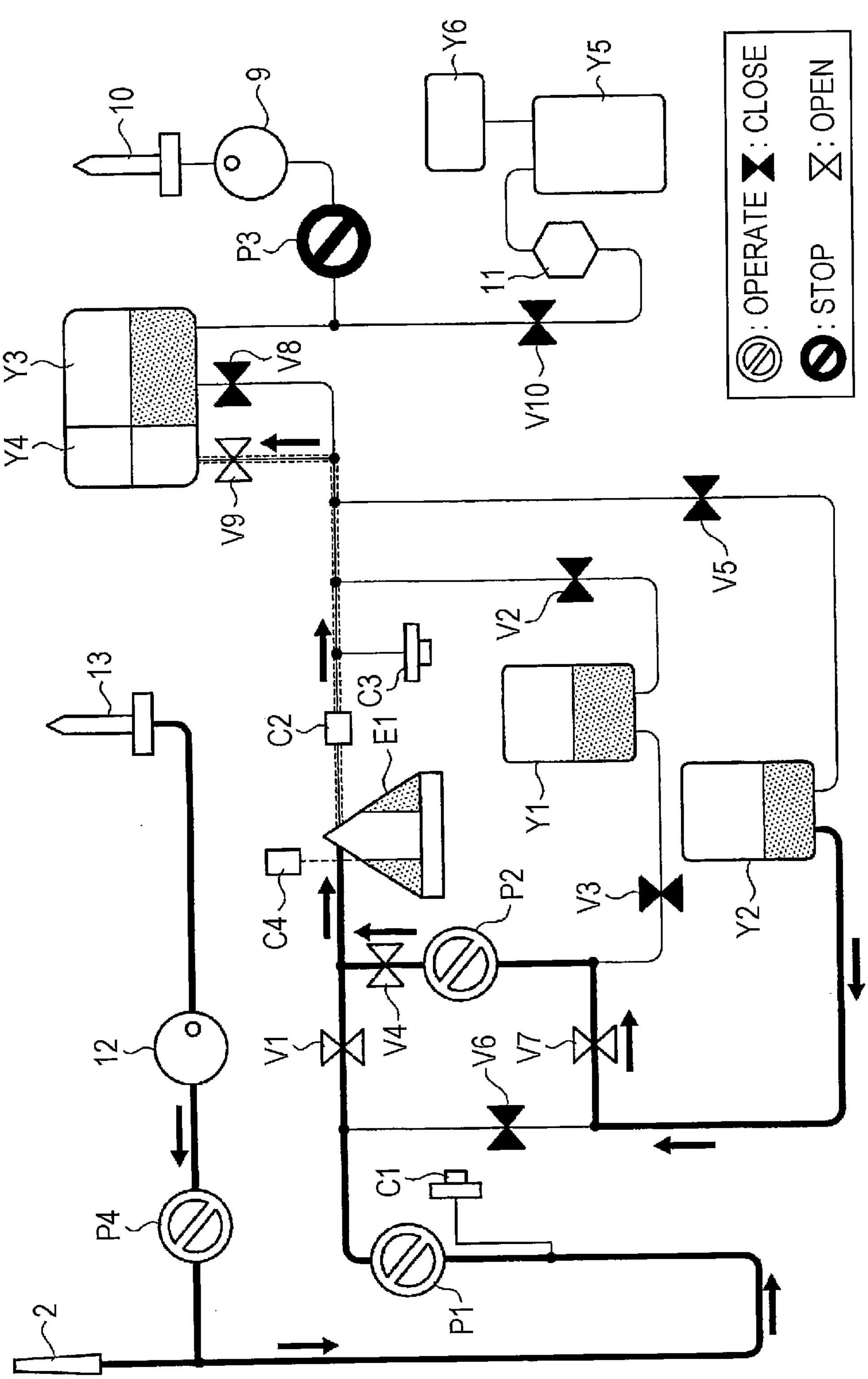
2
P4
12
13
P1
C1
V6
V7
V1
V4
P2
V3
C4
E1
C2
C3
Y1
Y2
V2
V5
V9
Y4
Y3
V8
V10
11
P3
9
10
Y5
Y6
: OPERATE
: CLOSE
: STOP
: OPEN

FIG. 15

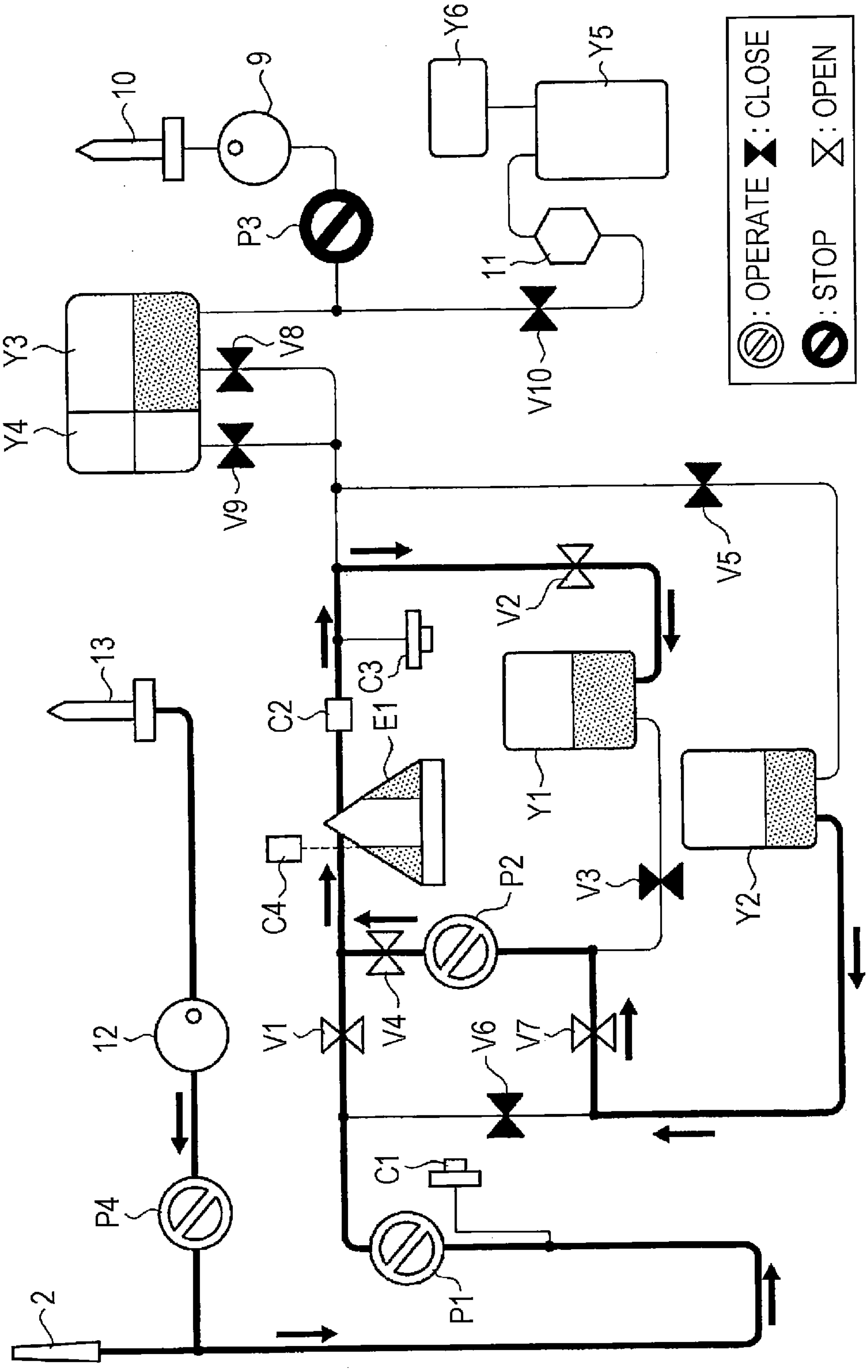
10
9
P3
Y6
Y5
11
V10
Y3
Y4
V8
V9
V5
V2
C3
C2
E1
Y1
13
C4
P2
V3
Y2
V4
V1
V6
V7
12
C1
P4
P1
2
: OPERATE
: CLOSE
: STOP
: OPEN

FIG. 16

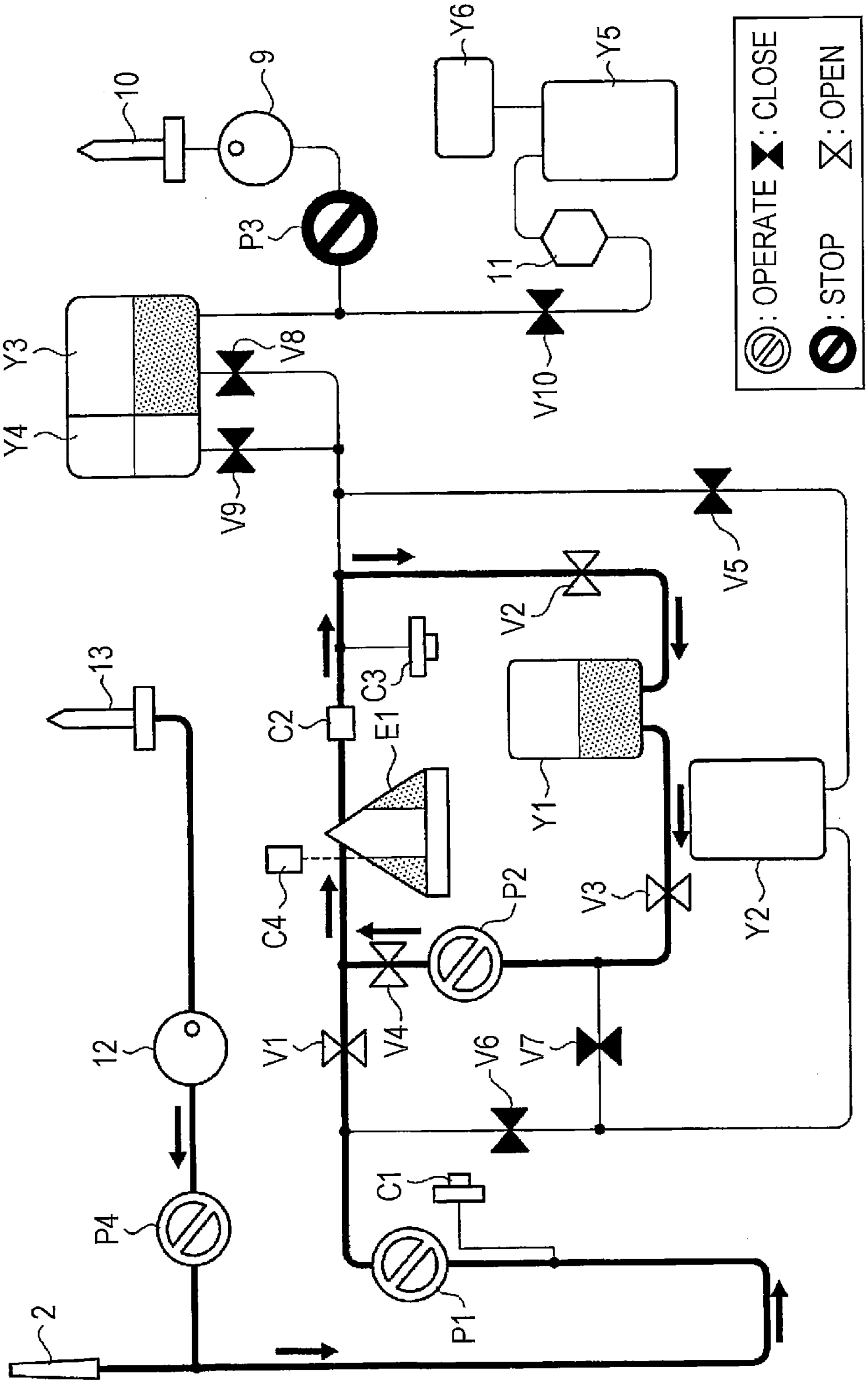
10
9
P3
Y6
Y5
Y3
Y4
V8
V9
V10
11
13
12
P4
2
P1
C1
C2
C3
C4
E1
V1
V2
V3
V4
V5
V6
V7
P2
Y1
Y2
: OPERATE
: CLOSE
: STOP
: OPEN

FIG. 17

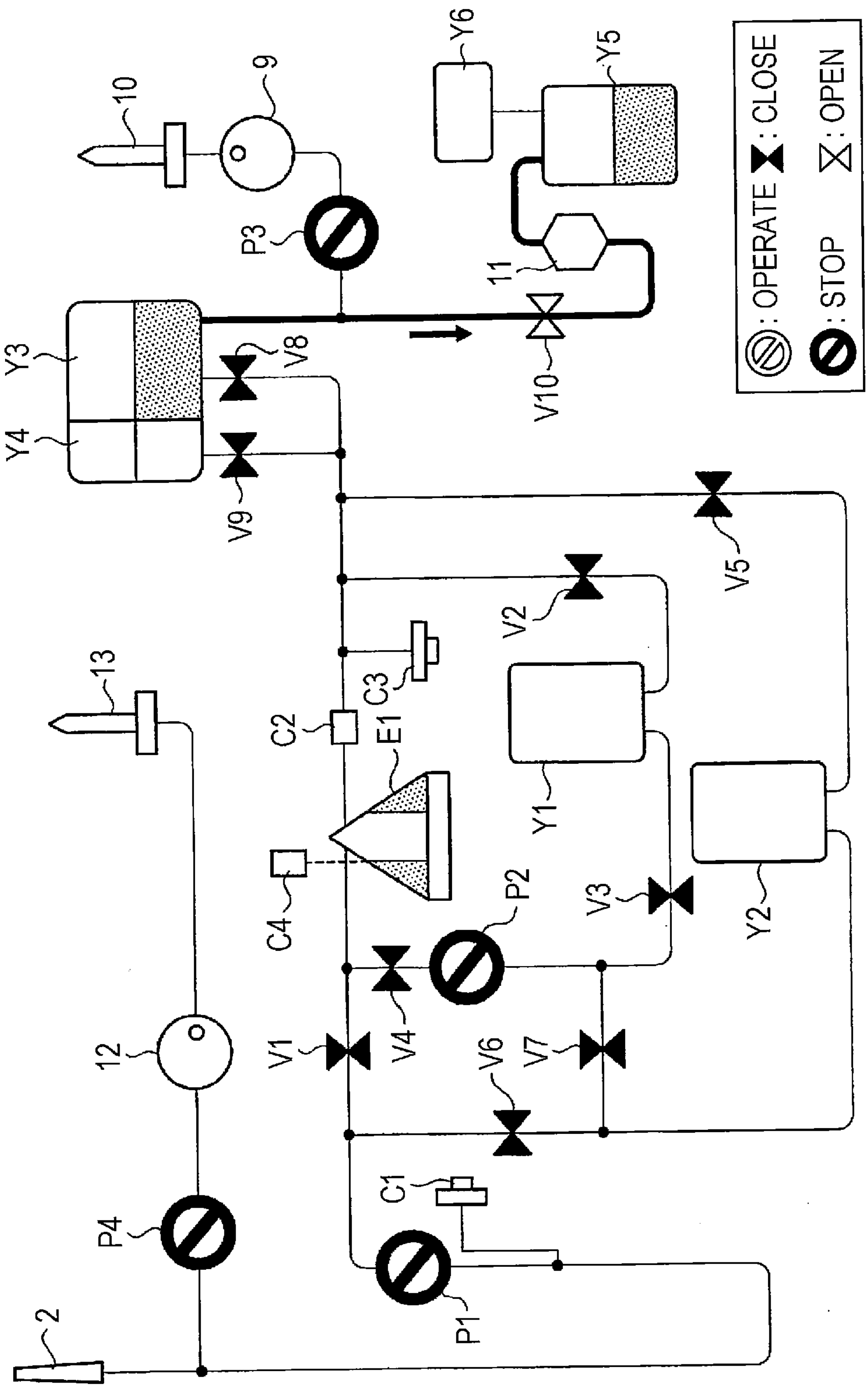
10
9
Y6
Y5
P3
11
Y3
Y4
V8
V9
V10
V5
V2
13
C3
C2
E1
Y1
Y2
C4
P2
V3
V4
V1
V6
V7
12
C1
P4
P1
2
: OPERATE
: CLOSE
: STOP
: OPEN

FIG. 18

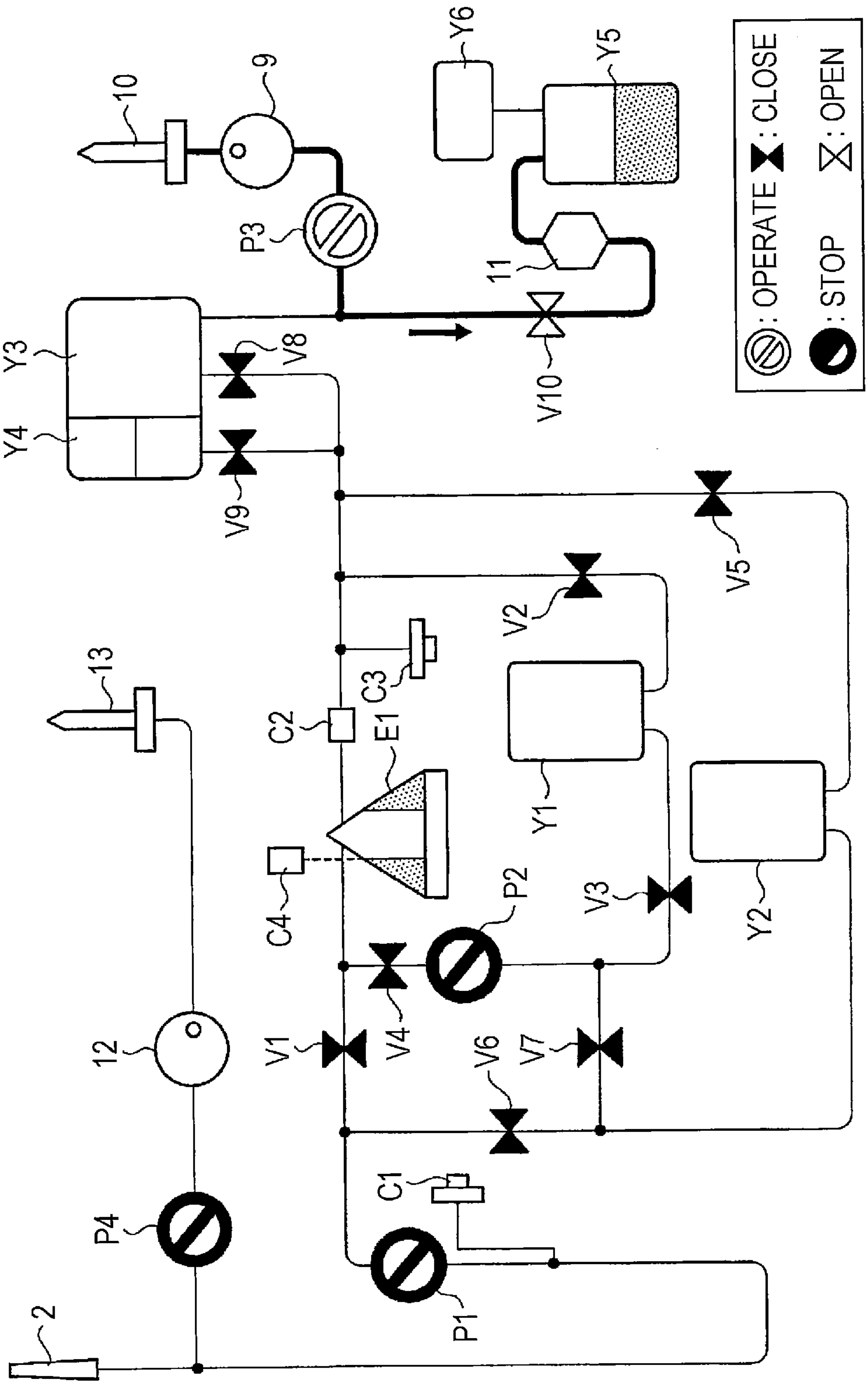
10
9
Y6
Y5
P3
11
Y3
Y4
V8
V9
V10
V5
V2
13
C3
C2
E1
Y1
C4
P2
V3
Y2
12
V1
V4
V6
V7
P4
C1
2
P1
: OPERATE
: CLOSE
: STOP
: OPEN

FIG. 19

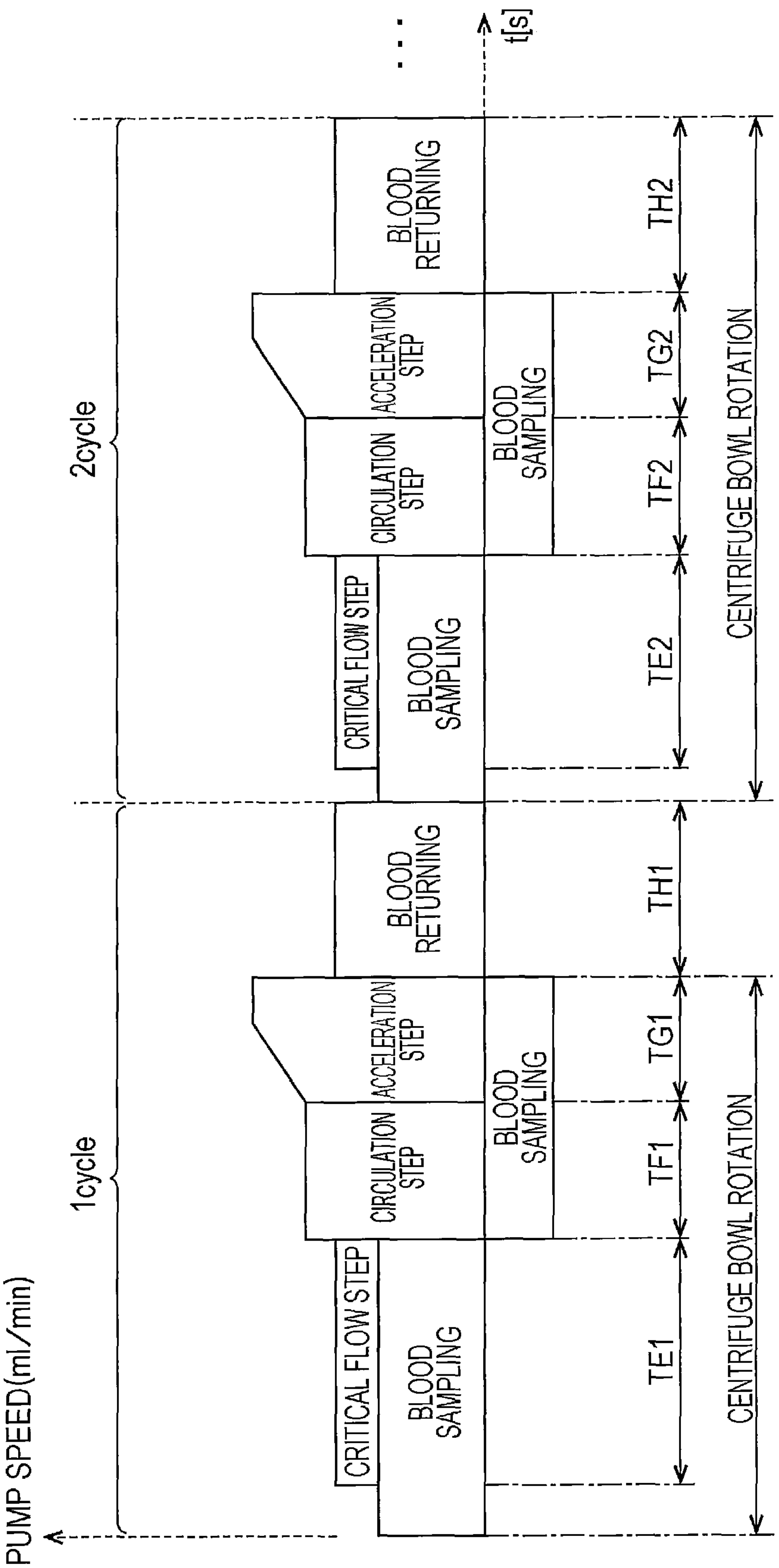
PUMP SPEED(ml/min)
t[s]
1cycle
2cycle
CRITICAL FLOW STEP
BLOOD SAMPLING
CIRCULATION STEP
ACCELERATION STEP
BLOOD RETURNING
TE1
TF1
TG1
TH1
TE2
TF2
TG2
TH2
CENTRIFUGE BOWL ROTATION

FIG. 20

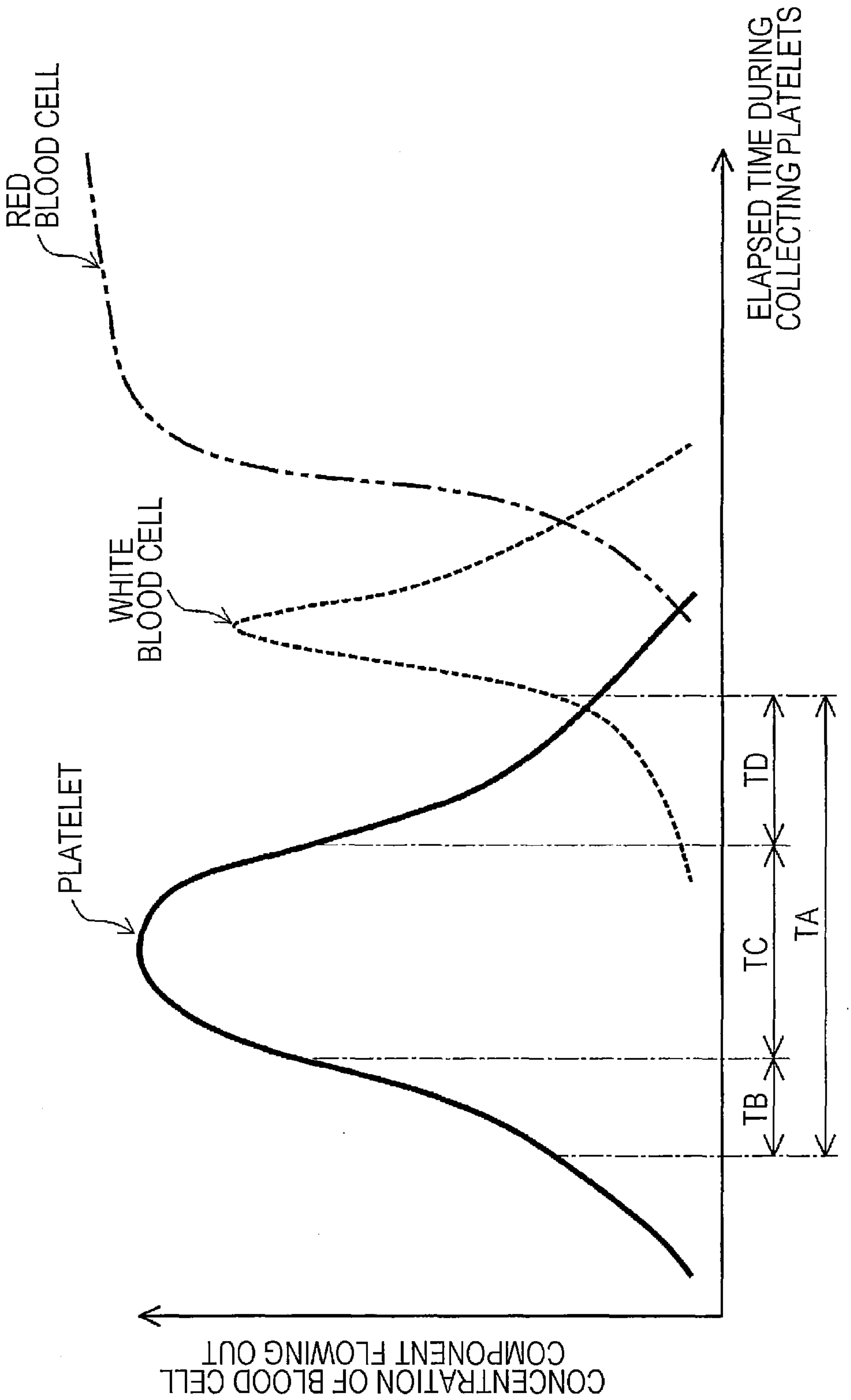
RED
BLOOD CELL
WHITE
BLOOD CELL
PLATELET
ELAPSED TIME DURING
COLLECTING PLATELETS
CONCENTRATION OF BLOOD CELL
COMPONENT FLOWING OUT
TA
TB
TC
TD

FIG. 21

| | ESTIMATED EXTRACORPOREAL CIRCULATION VOLUME [ml] | DRAWN VOLUME [ml] | |
|---|---|---|---|
| | | cycle 1 | cycle 2 |
| n | 31 | 31 | 31 |
| AVERAGE | 404 | 397 | 381 |
| σ | 28 | 24 | 23 |
| min | 365 | 362 | 341 |
| max | 486 | 466 | 438 |
| DATA | 401 | 395 | 376 |
| | 470 | 435 | 428 |
| | 413 | 389 | 368 |
| | 392 | 399 | 377 |
| | 439 | 437 | 409 |
| | 431 | 425 | 409 |
| | 377 | 365 | 346 |
| | 486 | 466 | 438 |
| | 393 | 384 | 364 |
| | 414 | 400 | 395 |
| | 398 | 389 | 376 |
| | ⋮ | ⋮ | ⋮ |

FIG. 22

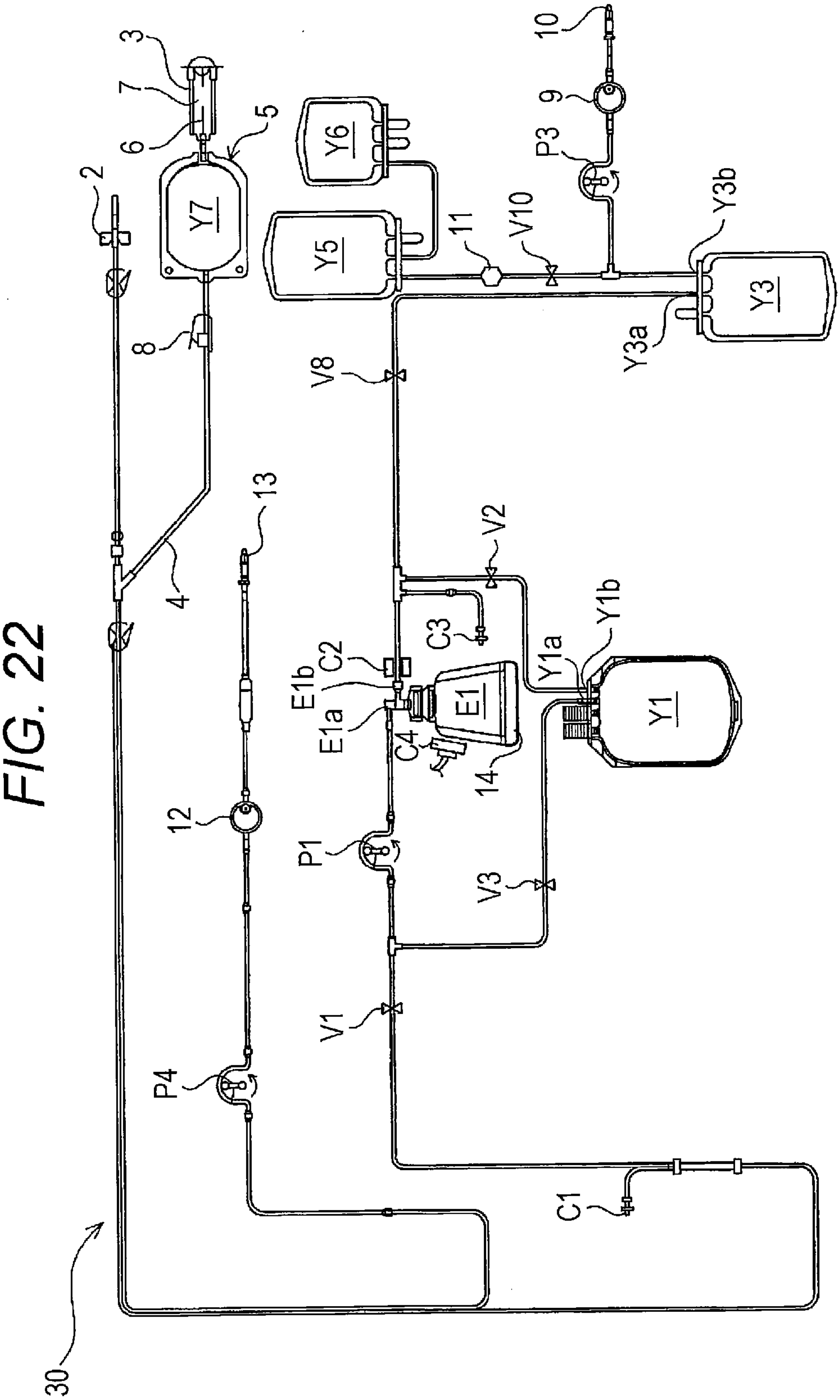

2
3
7
6
5
Y7
8
4
Y6
Y5
10
9
P3
11
V10
Y3b
Y3
Y3a
V8
13
V2
C3
C2
E1b
E1a
E1
C4
14
Y1a
Y1b
Y1
12
P1
V3
V1
P4
C1
30

FIG. 23

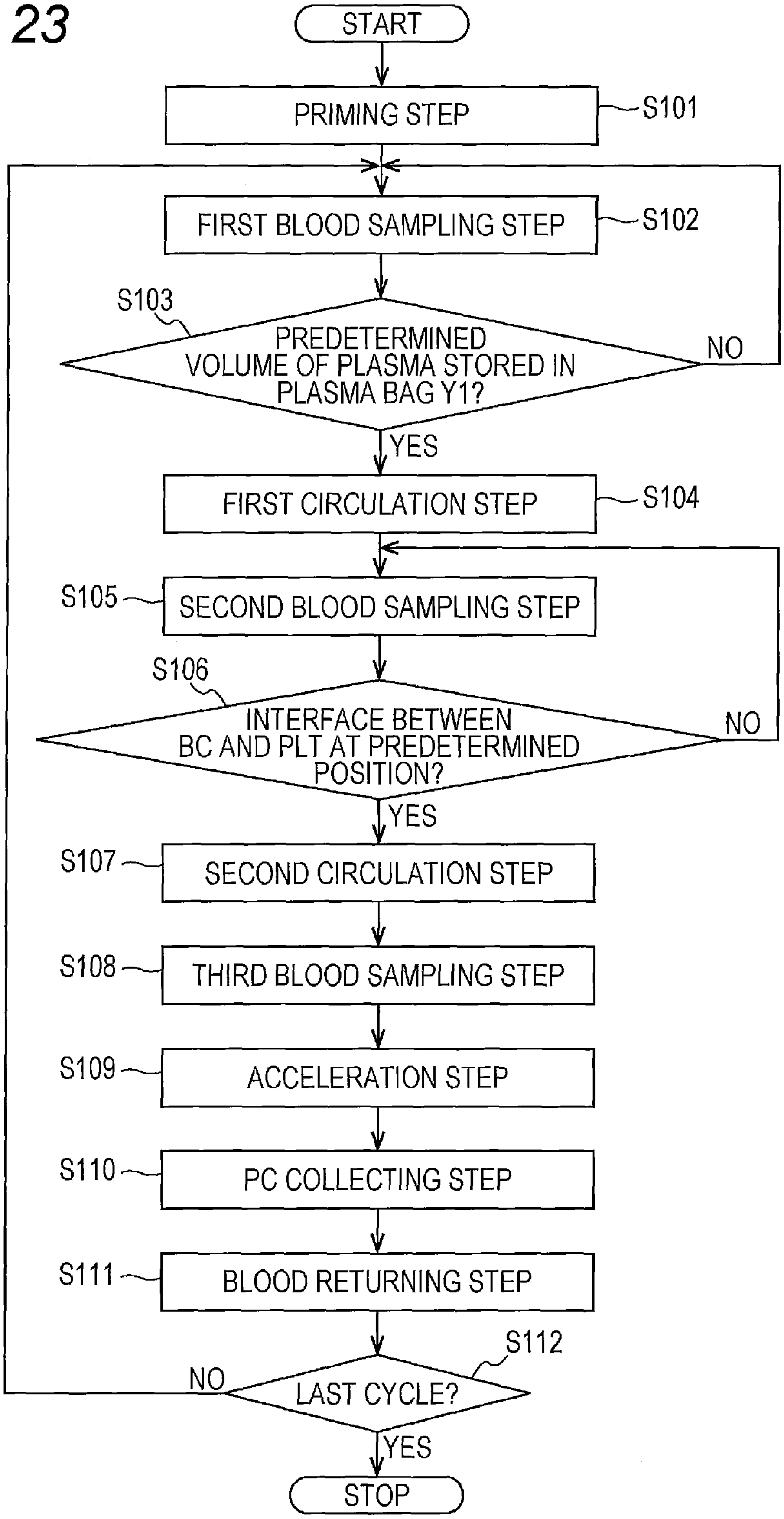
START
PRIMING STEP
S101
FIRST BLOOD SAMPLING STEP
S102
S103
PREDETERMINED VOLUME OF PLASMA STORED IN PLASMA BAG Y1?
NO
YES
FIRST CIRCULATION STEP
S104
S105
SECOND BLOOD SAMPLING STEP
S106
INTERFACE BETWEEN BC AND PLT AT PREDETERMINED POSITION?
NO
YES
S107
SECOND CIRCULATION STEP
S108
THIRD BLOOD SAMPLING STEP
S109
ACCELERATION STEP
S110
PC COLLECTING STEP
S111
BLOOD RETURNING STEP
S112
NO
LAST CYCLE?
YES
STOP

FIG. 24

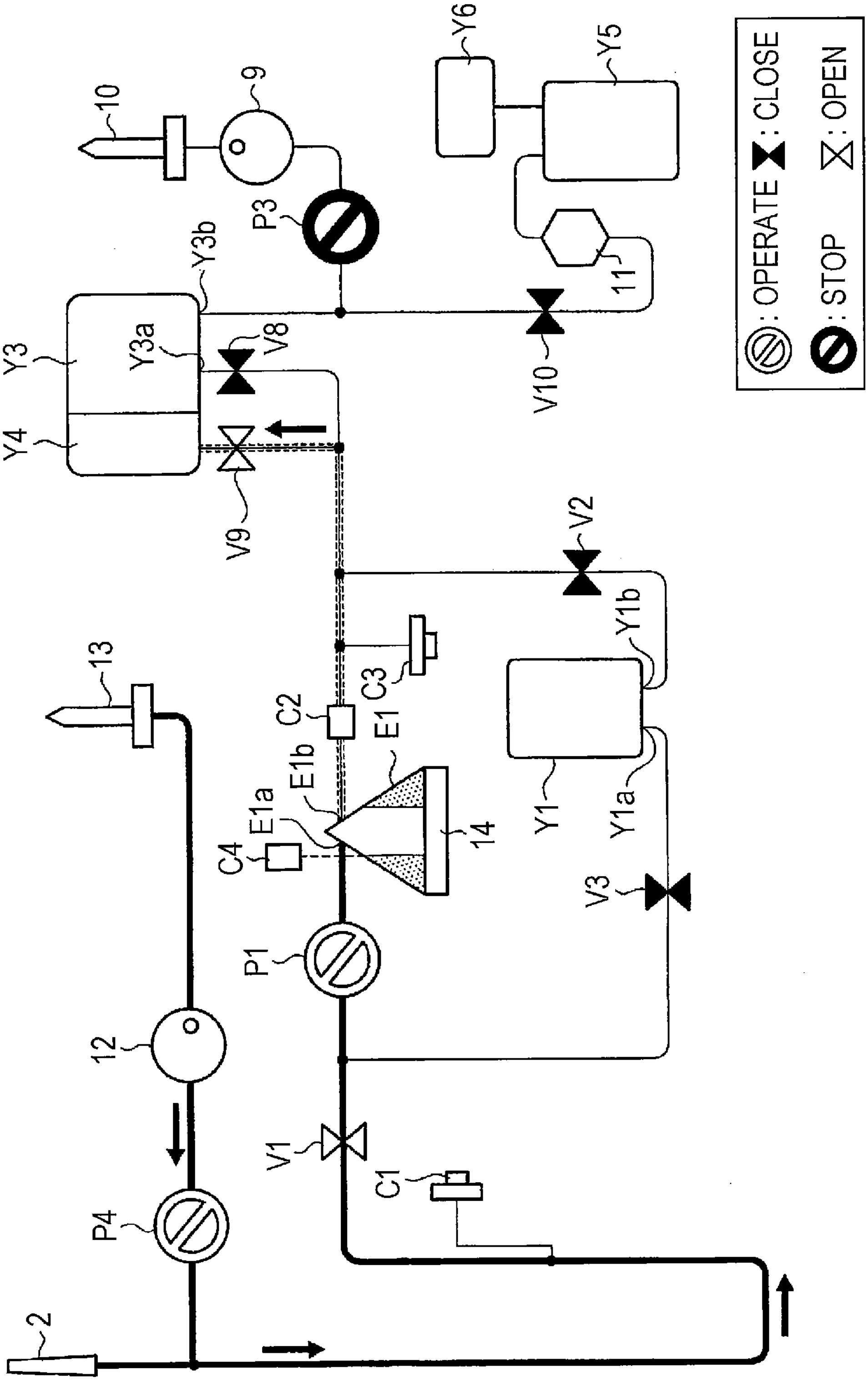
10
9
P3
Y3b
Y3
Y3a
V8
Y4
V9
V10
11
Y6
Y5
: OPERATE
: CLOSE
: STOP
: OPEN
13
V2
C3
Y1b
C2
E1
E1b
E1a
C4
14
Y1
Y1a
V3
P1
12
V1
C1
P4
2

FIG. 25

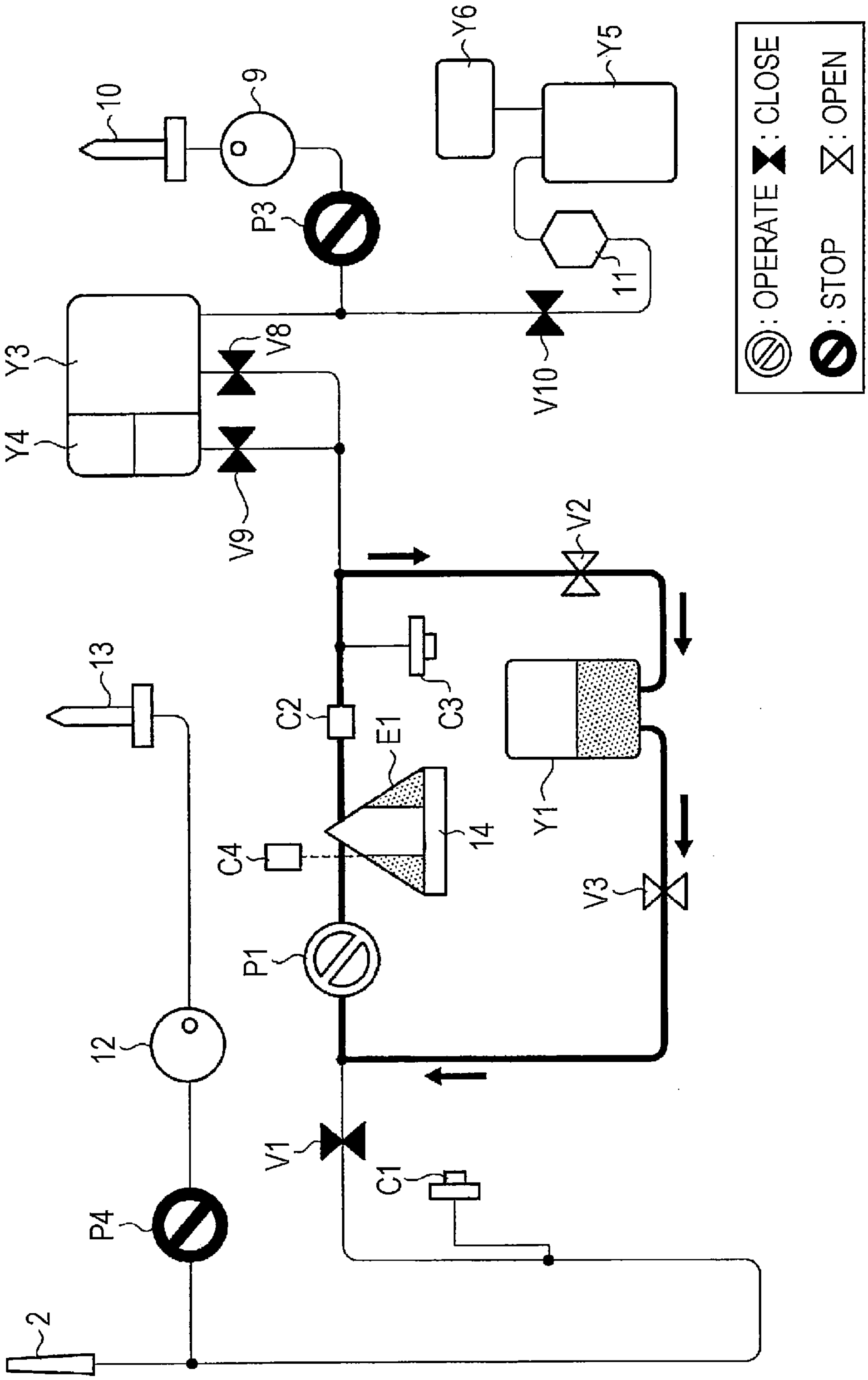

2
P4
12
13
V1
C1
P1
C4
14
E1
C2
C3
Y1
V3
V2
V9
V8
Y4
Y3
P3
9
10
V10
11
Y5
Y6
: OPERATE
: CLOSE
: STOP
: OPEN

FIG. 26

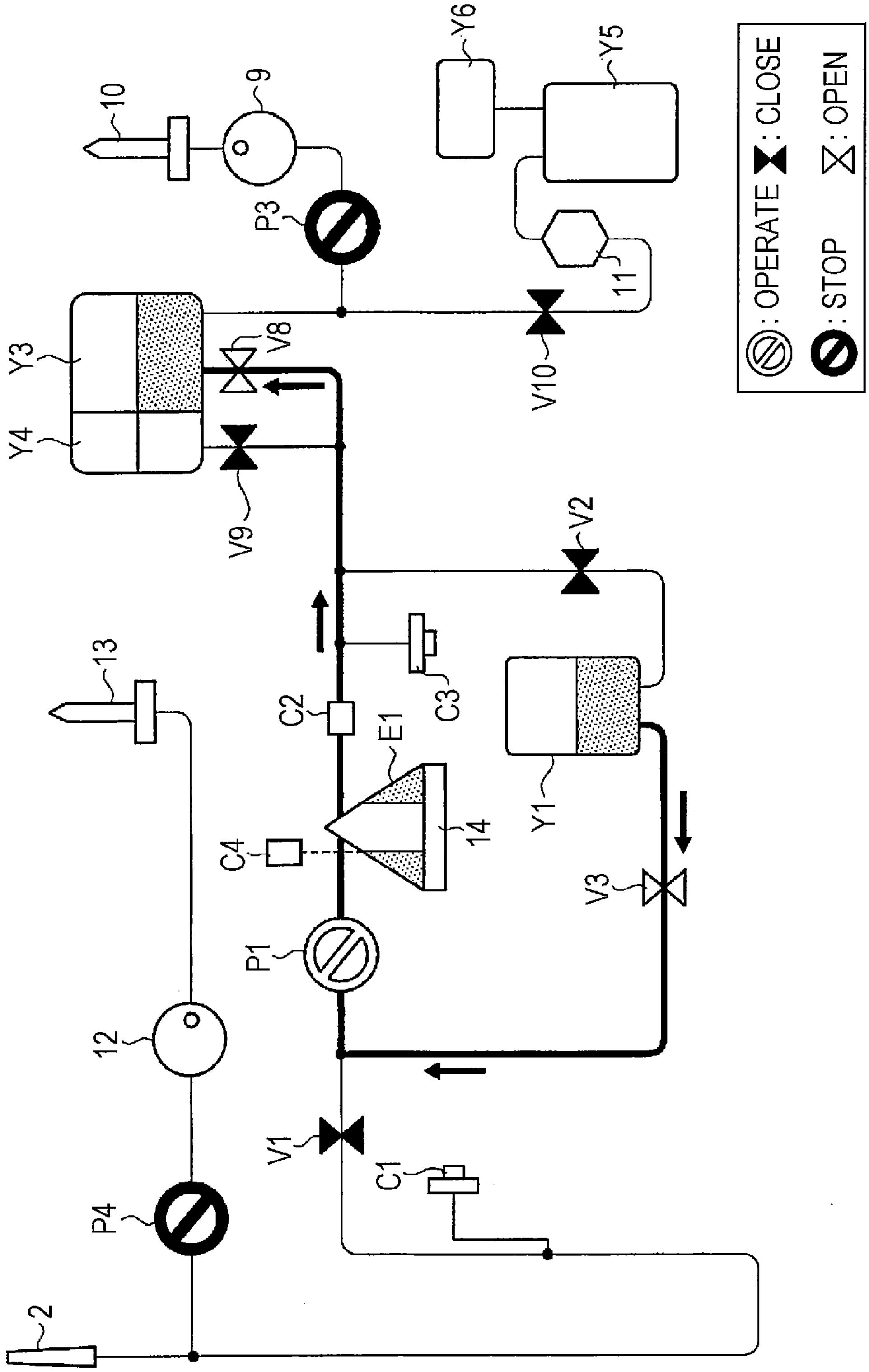
10
9
P3
Y6
Y5
11
Y3
Y4
V8
V9
V10
V2
13
C2
C3
E1
C4
14
Y1
V3
P1
12
V1
C1
P4
2
: OPERATE
: CLOSE
: STOP
: OPEN

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009226210 A
- US 6743192 B1